# EUROPEAN PATENT SPECIFICATION

(11) **EP 3 882 920 B1**
(45) Date of publication and mention of the grant of the patent: **11.12.2024**
(21) Application number: 20164701.3
(22) Date of filing: 20.03.2020
(51) Int. Cl.: G16H 40/40, G10L 15/00, G16H 40/67

(54) **PROCESSING DATA FROM A MEDICAL ANALYZER**
VERARBEITUNG VON DATEN AUS EINEM MEDIZINISCHEN ANALYSATOR
TRAITEMENT DE DONNÉES À PARTIR D'UN ANALYSEUR MÉDICAL

(43) Date of publication of application: 22.09.2021
(73) Proprietor: F. Hoffmann-La Roche AG, 4070 Basel (CH)
(72) Inventor: MARTIN, Stephen, 6343 Rotkreuz (CH); PAUN, Alexandra, 6343 Rotkreuz (CH); WINIARZ, Jakub, 6343 Rotkreuz (CH)
(74) Representative: Peterreins Schley

(56) References cited:
- WO-A1-03/043495
- WO-A1-2014/005106
- WO-A1-2018/236497
- WO-A1-2019/152966
- US-A1- 2006 259 265
- US-A1- 2017 017 538
- US-A1- 2019 035 489
- US-A1- 2019 088 351
- US-A1- 2019 311 812
- US-A1- 2020 049 724

## Description

### Technical Field

This disclosure relates to a computer-implemented method for operating a data processing agent for processing data from an automated analyzer of medical samples, and an associated apparatus, system, computer program element, and computer readable medium.

### Background

In clinical care environments, automated analyzers of medical samples can be used at, or near to, the point of care. Such automated analyzers are designated "Point of Care (POC) testing systems". The automated analyzers can communicate a variety of status messages containing information about their technical status with a central server, for example.

POC devices send a large number of regular device messages (events) to a POC IT data management system. As a consequence, the coherent monitoring of information concerning the status of a large number of POC devices can be challenging.

The documents US 2019/0088351 A1, US 2019/311812 A1, US 2006/259265 A1, WO 2014/005106 A1, WO 2019/152966 A1, WO 2018/236497 A1, WO 03/043495 A1 and US 2017/0017538 A1 concern aspects of patient monitoring.

### Summary

According to a first aspect, there is provided a computer implemented method for operating a data processing agent for processing data from an automated analyzer of medical samples as defined in independent method claim 1, to which the reader should now refer. Further aspects of an apparatus, a system, a computer program element, and a computer readable medium are also defined in the claims. Further embodiments are defined in the dependent claims.

An effect of the technique of the present aspects is that a large number of event messages sent by one or more automated analyzers informing about the technical condition of the one or more automated analyzers can be associated with annotation data provided from another source. Therefore, additional technical context about the technical condition of an automated analyzer can be gathered from diverse sources.

For example, an automated analyzer may issue a large number of over-temperature warnings, for which the reason is not immediately obvious. Following association with annotation data from another source, it may become clear that the automated analyzer has been left on the shelf above a radiator, rather that the device itself being defective. Such integration of contextual data enables remote personnel at a POC monitoring department or office to more easily explain technical deviations from normal operations of an automated analyzer.

Accordingly, a simple tracking mechanism is created for all troubleshooting activities that are shared between the team managing the automated analyzer. When a problem arises, a person responsible for a particular automated analyzer can add information about the technical condition of the device that can be associated with a group of automatically generated event messages from an automated analyzer. As an example, the combined group of automatically generated event messages from the automated analyzer and the annotation data provided from another source (such as a logbook) together form a single source of truth about the condition of a given automated analyzer.

This avoids the need to store notes on the technical condition of specific automated analyzers in disparate computer systems, or in paper form.

Another effect is that some automated analyzers repeatedly send the same message. For example, a hardware heartbeat signal, indicating that the automated analyzer hardware is operating correctly, may be sent to a system for automated analyzer management every second. Over time, this leads to the system for automated analyzer management receiving large number of messages which provide little contextual benefit about the technical condition of the automated analyzer. When displayed in a graphical user interface, the large number of hardware heartbeat signal messages create clutter and obscure messages that may be more significant (such as reporting a "battery low" alarm).

Accordingly, according to the present aspects, messages from the automated analyzer may be automatically grouped to enable a user of such graphical user interface to see the order and history whilst maintaining awareness of the fact that for example hundred messages of a given type are sent in a short period of time.

Certain terms will be used in this patent application, the formulation of which should not be interpreted to be limited by the specific term chosen, but as to relate to the general concept behind the specific term.

As used herein, the terms "comprises", "comprising", "includes", "including", "has", "having", or any other variation thereof, are intended to cover a non-exclusive inclusion.

The terms "patient sample" and "biological sample" refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semisolid biological material is rendered liquid by dissolving or suspending it with a suitable liquid medium. In some embodiments, the sample is suspected to contain a certain antigen or nucleic acid.

The term "automated analyzer of medical samples" as used herein encompasses any apparatus for obtaining a measurement value from a patient sample. For example, the analyzer measures light absorption, fluorescence, electrical potential or other physical or chemical characteristics of the reaction to provide the measurement data. Often such patient samples are treated before analytical testing is done. Blood sampled from a patient is e.g. centrifuged to obtain serum or treated with anti-coagulants to obtain plasma. An "automated analyzer of medical samples" may be configured so as to be usable in the vicinity of a patient ward, in which case it is often referred to as a "Point of Care (POC) device". However, the techniques discussed herein are not limited to POC devices and may be applied to many types of laboratory analysis system.

Analytical testing by an analyzer has the goal to determine the presence and/or concentration of an analyte in a patient sample. The term "analyte" is a general term for substances for which information about presence and/or concentration is intended. Examples of analytes are e.g. glucose, coagulation parameters, endogenic proteins (e.g. proteins released from the heart muscle), metabolites, nucleic acids and so on.

The term "patient health parameter" as used herein encompasses any aspect of a patient's physiology that is measurable or indicated by an analysis of a patient sample for one or more analyte.

Therefore, an automated analyzer of medical samples analyzer may be used in a point of care environment, such as (but not limited to) blood glucose testing, coagulation testing, blood gas and electrolytes analysis, urinalysis, cardiac markers analysis, hemoglobin diagnostics, infectious disease testing, cholesterol screening or nucleic acid testing NAT. Results may be viewed directly on the POC analyzer(s) or may be sent to the POCT system and displayed in a Laboratory Information System with central lab results, or alongside imaging results in a Hospital Information System.

The term "analytical data" as used herein encompasses any data that is descriptive of a result of a measurement of one or more patient health parameter(s) performed by a POC analyzer of the biological sample that has been analyzed. In case of a calibration the analytical data comprises the calibration result, i.e. calibration data. In particular, the analytical data comprises an identifier of the patient sample for which the analysis has been performed and data being descriptive of a result of the analysis, such as measurement data.

The term "Point of Care" POC or "Point of Care environment" as used herein is defined to mean a location on or near a site of patient care where medical or medically related services such as medical testing and/or treatment are provided, including but not limited to hospitals, emergency departments, intensive care units, primary care setting, medical centers, patient homes, a physician's office, a pharmacy or a site of an emergency.

The term "point of care testing" POCT as used herein encompasses analysis of one or more patient sample(s) in a point of care environment. POCT is often accomplished through the use of transportable, portable, and handheld instruments, but small bench analyzers or fixed equipment can also be used when a handheld device is not available- the goal being to collect the patient sample and obtain the analytical data in a (relatively) short period of time at or (relatively) near the location of the patient.

POCT is performed using various POC analyzers such as (but not limited to) analyzers for glucose, coagulation, blood gas, urinalysis, cardiac and molecular testing. Results may be viewed directly on the POC analyzer(s) or may be sent to the POCT system and displayed in a Laboratory Information System with central lab results, or alongside imaging results in a Hospital Information System.

The term "portable computing device" encompasses any electronic appliance that can be moved easily from one location to another, in particular any handheld battery powered mobile appliance, including but not limited to a cellular telephone, a satellite telephone, a pager, a personal digital assistant ("PDA"), a smartphone, a navigation device, a smart book or reader, a combination of the aforementioned devices, a tablet computer or a laptop computer.

The term "communication network" as used herein encompasses any type of wired or wireless network, including but not limited to a WIFI, GSM, UMTS or other wireless digital network or a wired network, such as Ethernet or the like. For example, the communication network may include a combination of wired and wireless networks.

The term "server" encompasses any physical machine or virtual machine having a physical or virtual processor, capable of accepting requests from and giving responses accordingly. It shall be clear to a person of ordinary skill in the art of computer programming that the term machine may refer to a physical hardware itself, or to a virtual machine such as a JAVA Virtual Machine (JVM), or even to separate virtual machines running different Operating Systems on the same physical machine and sharing that machine's computing resources. Servers can run on any computer including dedicated computers, which individually are also often referred to as "the server" or shared resources such as virtual servers. In many cases, a computer can provide several services and have several servers running. Therefore the term server shall encompass any computerized device that shares a resource to one or more client processes.

The term "server interface" encompasses any hardware-, firmware- and/or software- based module operable to execute program logic to allow communication with an external entity (such as a server or another interface).

The term "data processing agent" refers to a software module executing on one or more computing devices, such as a server, that is able to receive automated analyzer status data from a point of care device, and annotation data from a user or operator, and associate the automated analyzer status data and the annotation data. The "data processing agent" may be implemented on a single server, or multiple servers, and/or an internet-based "cloud" processing service such as Amazon AWS (TM) or Microsoft Azure (TM). The "data processing agent" may be hosted on a virtual machine.

The term "user interface" encompasses any suitable piece of software and/or hardware for interactions between an operator and a machine, including but not limited to a graphical user interface for receiving as input a command from an operator and also to provide feedback and convey information thereto. Also, a system / device may expose several user interfaces to serve different kinds of users/ operators.

In the field of bedside testing or point of care testing the testing is done on patients typically by nurses, medical staff or doctors but also pharmacists who are collectively called "operator(s)" herein. However, anyone who possesses the required certification may be an operator. A point of care coordinator POCC may be at the same time an operator of POC analyzer(s) and also an operator of POC analyzer(s) may be at the same time a point of care coordinator POCC and thus user of portable computing device(s).

The term "certification" as used herein encompasses any form of confirmation of certain characteristics (such as training and/or examination and/or educational background and/or accreditation) of an operator. In particular a certification as disclosed herein is not restricted to embodiments which are formally titled "certification" or physical embodiments (such as a printed certification) having a related title. According to embodiments of the disclosed system/ method, certification(s) are provided by an entry of an operator on list(s) of certified operators allowed to perform a job/ task / workflow or step of a workflow using one or more of the POC analyzers. The certification(s) according to embodiments of the disclosed system/ method may be permanent and/or time-restricted certifications, meaning that the certification corresponding to an operator becomes invalid after a certain period of time. After a certification becomes invalid, the respective operator needs to become certified again (by taking a training and/or passing a (re)examination), otherwise that operator may no longer use the respective POC analyzer(s) or certain features / functions thereof.

The term "system certification" as used herein encompasses a certification stored on a server. According to embodiments, the system certification relates to all POC analyzers of the POC testing system. According to further embodiments, the system certification relates to one or more POC analyzers of a certain type, class, namely POC analyzers having at least one common characteristic. Examples of common characteristics of one or more POC analyzers are: POC analyzers being capable of performing the same or similar analyses of patient sample(s); POC analyzers requiring the same or similar operator training/ examination / certification; POC analyzers from one manufacturer; POC analyzers at the same healthcare facility, etc.

The term "analyzer certification" as used herein encompasses any certification stored on a POC analyzer. According to some embodiments, the analyzer certification stores any kind of representation of the operators (such as a list of operator identifiers) authorized to perform at least one job/ task / workflow or step of a workflow using that particular POC analyzer, in particular operators authorized to analyze one or more patient sample(s) using that particular POC analyzer. According to certain embodiments, each analyzer certification is specific to one particular POC analyzer. According to further embodiment(s), each analyzer certification is specific to one or more POC analyzers of a certain type, class. According to even further embodiment(s), the analyzer certification(s) may be identical to the system certification(s), but stored locally on the POC analyzer(s).

The term "automated analyzer status data" refers to data that may be transmitted by, for example, a POC analyzer that provides updates on the operating condition of the POC. The automated analyzer status data may generated by the automated analyzer in response to a condition inside the automated analyzer. The "automated analyzer status data" is transmitted over a communications network and may comprise a wide range of information about the POC device, for example, but not limited to: temperature or battery state of the analyzer, status of lids or doors, status of electromechanical components such as motors, lasers, or photometers, amount of free memory, software or firmware installation version, total use duration, data relating to the status or sub steps of individual analysis runs, location of the analyzer, network parameters of the data such as connection data rate, certification status of operators, a POC device error code, and the like.

The term "annotation data" refers to contextual information concerning the operation of the device provided by an operator. Traditionally, such data is collected in a paper logbook, or diary, owned by the operator or the POCT team. As such, the annotation is traditionally unstructured text. A POC device operator may have tens or hundreds of individual devices in their care, and unstructured comments concerning the operation of one, or a group of analyzers are usually made in a paper logbook for the sake of convenience and speed. Therefore, annotation data is unstructured data concerning the condition of one or more automated analyzer. The annotation data is not limited to text. For example, it could comprise a video, photo, or audio sample of the operation of an analyzer to illustrate the malfunction of a mechanical part, or an unexpected message on the screen of the POC device.

The term "associating one or more of the items of automated analyzer status data with one or more of the items of analyzer data" means that the data processing agent provides a logical link between at least one message output by a POC testing device, and at least one portion of annotation data provided by a system user, for example. A logical link may be an entry in a database that connects two records (one of annotation data, one of automated analyzer status data). Optionally, the association may be performed by correlating one or more items of automated analyzer status data with one or more of the items of analyzer data. Optionally, the association may be performed by prompting a user to add a connection between automated analyzer status data and one or more of the items of annotation data. Optionally, the association may be performed via a GUI by enabling a user to input annotation data against a displayed representation of an item of automated analyzer status data.

### Description of the Drawings

**FIG. 1** schematically illustrates a system for automated analyzer management.
**FIG. 2** schematically illustrates a computer implemented method for operating a data processing agent for processing data from an automated analyzer of medical samples.
**FIG. 3** schematically illustrates an example of a Point of Care (POC) device.
**FIG. 4** schematically illustrates an example of a server configured to host a data processing agent.
**FIG. 5** schematically illustrates messaging in a use case in the system for automated analyzer management.
**FIG. 6A** schematically illustrates an example of a low-battery messaging scenario in a system for automated analyzer management.
**FIG. 6B** schematically illustrates an example of a temperature anomaly messaging scenario in a system for automated analyzer management.
**FIG. 7** schematically illustrates an example of a correlator for associating items of automated analyzer data with annotation data.
**FIG. 8** schematically illustrates an example of correlating automated analyzer data with annotation data using a correlation rule set.
**FIG. 9A** illustrates a first example of a GUI, in use.
**FIG. 9B** illustrates a second example of the GUI, in use.
**FIG. 9C** illustrates a third example of the GUI, in use.
**FIG. 9D** illustrates a fourth example of the GUI, in use.

Note: The figures are not drawn to scale, are provided as illustration only and serve only for better understanding but not for defining the scope of the invention. No limitations of any features of the invention should be inferred from these figures.

### Detailed Description

POC analyzers (also known as automated analyzers of medical samples) are commonly managed by a server, and in particular, a hardware management server, also called Point of Care Data Management System (POC-DMS). Such a server provides connectivity for POC analyzers and management of test results, operators, quality controls, and analyzers.

Management of POCT is challenging - there can be dozens of sites, hundreds of POCT devices/kits, and thousands of operators to manage to assure quality of testing. One challenge in developing a strategy to manage POCT usually how a team of interdisciplinary POC management operators including the laboratory, physicians, and nurses handle the large amount of feedback data generated by the POC devices. The POC team should usually hold the responsibility for determining the test menu, selecting technologies, establishing policies and procedures, ensuring training and regulatory compliance, and providing advisory assistance to the end operators of POC technologies.

POC analyzers send a large amount of data comprised of regular device messages (events) to a system for automated analyzer management (POC-DMS). The device messages enable the automated analyzer to inform users about device usage, system warnings, system events, errors, or other events relating to the technical condition of the POC analyzer.

Point-of-care coordinators (POCCs) are responsible for managing POC devices. Typically, they keep detailed notes and comments about the handling of each device with a large group of devices, and for example who has used the devices, and the devices have been cleaned, or sent for repairs. These detailed notes are typically in the form of unstructured text (for example, in handwritten logbook or diary, or a rudimentary computer text diary).

Until the present time, the two sources of information about the condition of an automated analyzer (POC device) in a group of automated analyzers have been kept separate, either because the logbook notes were kept in paper form and never transcribed, or because the logbook notes were stored in software isolated from the POC-DMS.

It would, therefore, be desirable to use the unstructured logbook notes to enrich, and provide technical context to, the automated event messages provided by the automated analyzer. Accordingly, it is proposed to provide an interface that performs one or more of the following:
(1) combining automated analyzer messages and POCC notes into an aggregated data record and/or display.
(2) Aggregating multiple automated analyzer messages of the same type into an aggregated data record and/or display to enhance clarity of the displayed records.
(3) Enabling a user to add comments to received automated analyzer messages.

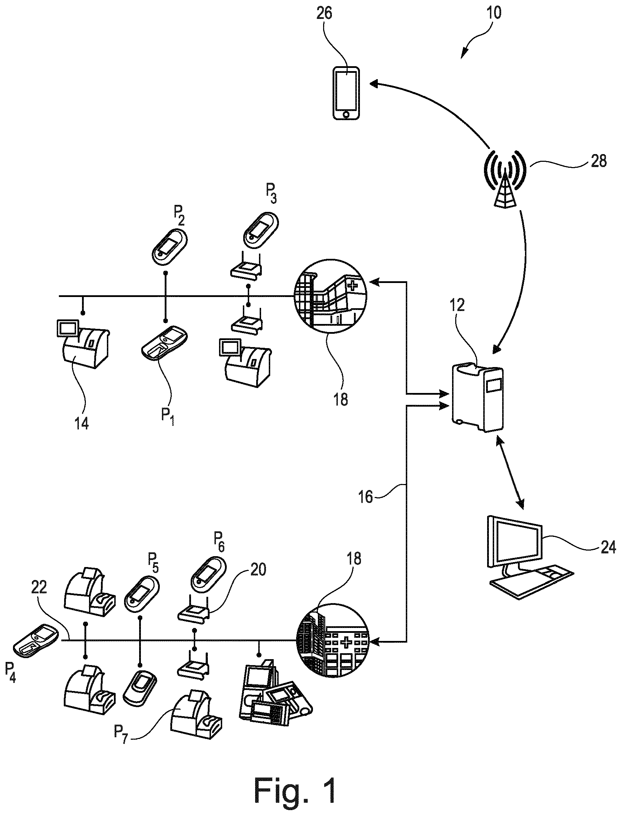

FIG. 1 schematically illustrates a system for automated analyzer management. The system for automated analyzer management comprises one or more automated analyzers (POC devices) P1 to P7, a portable computing device 26 (such as a smartphone), and a server 12 communicatively connected by a communication network 16. The server 12 may, in an example, host a data processing agent according to the first aspect. In other examples, the data processing agent may be a hosted by a cloud computing service distributed over a plurality of servers and computing devices. In particular, the communication network 16 is configured to communicatively connect the one or more automated analyzers P1 to P7.

The communication network 16 may, for example, comprise one or more of a local area network LAN provided over, for example, an ethernet network, a Wi-Fi network, and/or a wide area network WAN such as the Internet. The communications network may comprise a Mobile Telecommunications network such as a 3G, 4G, or 5G system 28, and/or a hospital PACS network.

Optionally, the communication network 16 may connect the server 12 directly to the automated analyzers (POC devices) P1 to P7 (not illustrated).

Optionally, the communication network 16 interfaces with an internal communications system 22 of a hospital 18. The internal communications system 22 may be considered to be an intranet, for example. A firewall and other security measures known to a person skilled in the art may be placed in between the internal communications system 22 and the communications network 16 to ensure security and confidentiality. The automated analyzers P1 to P7 may communicate with a data processing agent executing on the server 12, for example, by communicating via the internal communications system 22 and the communication network 16.

The automated analyzers P1 to P7 are provided and configured for analyzing one or more patient samples in order to measure one or more patient health parameters. According to disclosed embodiments, automated analyzers P1 to P7 may include transportable, portable, and hand-held instruments, but also small bench analyzers or fixed equipment 14 as well. For example, the automated analyzers may comprise (but not be limited to) blood glucose testing, coagulation testing, blood gas and electrolyte analysis, urine analysis, cardiac marker analysis, haemoglobin analysis, infectious disease testing, cholesterol screening or nucleic acid testing. Several functional and/or operational aspects of the automated analyzers P1 to P7 are configurable or customizable using one or more analyzer parameters.

The analyzers P1 to P7 are located in a hospital ward 18, for example. The fixed equipment 14 may be located in a hospital pathology laboratory, for example

In order to identify a particular automated analyzer P1 to P7, each is provided with an analyzer identifier, in particular in the form of an identifier tag such as a barcode and/or an RFID tag or a serial number. Optionally, such identifiers may be associated with an entry in a database of the system for automated analyzer management.

The analyzers P1 to P7 are, for example, configured to transmit automated analyzer status data (event messages) from the analyzers to the server 12 over the communications network 16.

According to a first aspect there is provided: a computer implemented method for operating a data processing agent for processing data from an automated analyzer of medical samples comprising:
- receiving 32 one or more items of automated analyzer status data transmitted from one or more automated analyzers in a medical sample analysis system to the data processing agent via a communications network, wherein the automated analyzer status data is generated by the one or more automated analyzers in response to an automated analyzer condition detected by a respective automated analyzer;
- receiving 34 one or more items of annotation data at the data processing agent;
- associating 36 one or more of the items of automated analyzer status data with one or more of the items of annotation data using the data processing agent to thus generate an automated analyzer system data record; and
- outputting 38 the automated analyzer system data record from the data processing agent, wherein the automated analyzer system data record comprises one or more items of automated analyzer device status data associated with one or more of the items of annotation data.

According to an embodiment, the method further comprises detecting, at the data processing agent, that the one or more items of automated analyzer status data should be supplemented with further data based on an annotation rule set.

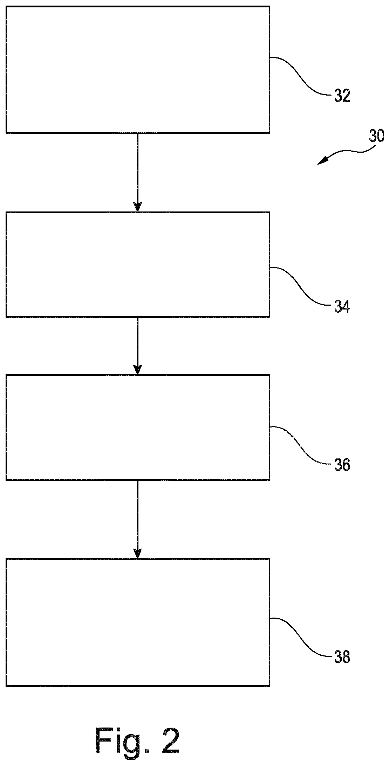

FIG. 2 schematically illustrates the computer implemented method for operating a data processing agent for processing data from an automated analyzer of medical samples.

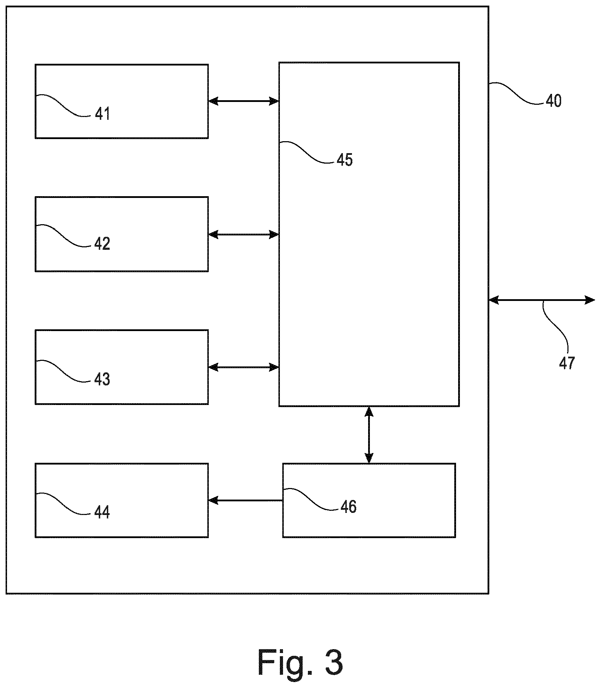

**FIG.** 3 schematically illustrates an example of an automated analyzer device (such as a POC device).

The illustrated example of a generic automated analyzer 40 comprises a power supply 46 configured to provide power to the automated analyzer 40. The power supply may be, for example, a lithium ion battery enabling the automated analyzer 40 to be portable. The power supply provides electrical energy to the other elements of the automated analyzer 40 comprising a sensor device 41, an electromechanical subassembly 42, a specimen processing section 43, and an analysis unit 44. A control and communication subsystem 45 interfaces with the previously listed modules. A communications link 47 enables data transfer to and from the automated analyzer 40 via a communications network 16.

The sensor device 41 may, for example, comprise a photometer for measuring optical transfer characteristics through a fluid sample, although many other types of sensor could be used depending on the application of the automated analyzer 40.

The electromechanical subassembly 42 is configured to receive sample ampoules or cassettes and load them into a specimen processing section 43 so that they can be analyzed by the sensor device 41. Following analysis, the electromechanical subassembly 42 may eject the sample ampoules or cassettes.

The specimen processing section 43 may perform pre-analysis functions such as agitation or heating of the sample to a required analysis temperature.

The analysis unit 44 may receive data from the sensor device 41 comprising a characterization of a specimen contained in the specimen processing section 43. The analysis unit 44 may perform one or more data processing operations on the data from the sensor device 41. For example, the analysis unit 44 may ensure that the result from the sensor device 41 is within expected boundaries.

Following analysis, the analysis unit 44 may transmit data from the sensor device 41 via the communications and control unit 45 to the system for automated analyzer management via the communications network 16, and eventually to a data processing agent hosted on, for example, the server 12.

A skilled person will appreciate that the description of a generic automated analyzer 40 is provided for illustrative purposes, and that practical automated analyzers may comprise fewer or more modules and functionalities.

The automated analyzer 40 may be configured to generate automated analyzer status data, and to transmit the automated analyzer status data over the communications network 16 to a data processing agent. Each module of the automated analyzer 40 may be configured to generate different types of automated analyzer status data (event messages, event data).

For example, the power supply 43 may generate the automated analyzer status data "batt_lo_10%" to indicate that the power supply 43 only has 10% of its capacity remaining.

For example, the power supply 43 may generate the automated analyzer status data "batt_shutdown" to indicate that the power supply 43 only has shut down the battery owing to a battery fault, or having run out of battery power.

For example, the electromechanical subassembly 42 may generate the automated analyzer status data "motor_PCB_HB" as a repetitive "heartbeat signal" indicating that it is continuously functional.

For example, the sensor device 41 may generate a photometer printed circuit board "heartbeat" signal. In addition the sense device 41 may generate the automated analyzer status data "photometer_clean_warn" indicating that the on-board LED and/or laser requires cleaning.

For example, the specimen processing section 43 may report the automated analyzer status data "door_jam" to signal that a sample handling door of the automated analyzer has not closed to contain the sample securely.

For example, the control and communications unit 45 may generate automated analyzer status data in the form of a "temp_hi_90%" signal indicating that the operating temperature of the automated analyzer is approaching an unsafe temperature at which inaccurate results may be provided. For example, the control and communications unit 45 may generate automated analyzer status data in the form of a "temp_auto_shutdown" signal indicating that the automated analyzer 40 has been switched off owing to an excessive temperature.

The control and communication unit 45 may also transmit automated analyzer status data as sequence of analysis states ("scan_barcode", "report_barcode", "assay_loaded", "test_result") to enable status of individual tests to be tracked.

The control and communication unit 45 may provide automated analyzer status data reporting on software configuration aspects of the automated analyzer 40 such as the state of internal memory, a current software or firmware version, and security parameters such as the success or failure of passwords, and networking aspects such as reporting network configurations settings, and optionally network uptime and downtime.

Optionally, the automated analyzer status data is time stamped by the control and communication unit 45 to an accuracy of 10 seconds, one second, 0.1s, 0.01s, or an even higher accuracy as enabled by, for example, the ethernet time protocol or network time protocol. This enables the data processing agent in the server 12 to reconstruct the sequence of received automated analyzer status data relative to the time that an event occurred triggering the generation of the automated analyzer status data.

Of course, the control and communication unit 40 may generate more complicated automated analyzer status data comprising concatenated groups of individual event messages based on rules contained in the control and communications unit 40 of the automated analyzer.

Optionally, the automated analyzer status data may be concatenated with test result data obtained from the analysis of a patient sample.

The automated analyzer status data is transmitted as a data packet encapsulated according to the communication system 16 used in the system for automated analyzer management, as known by persons skilled in the art. The data packet may comprise any necessary arrangement of header information to enable reliable routing of the automated analyzer status data to the data processing agent. The data packet may comprise only one bit of information (for example in the case of a heartbeat flag). Alternatively, the data packet may comprise a large amount of information (for example several kilobytes or megabytes. The control and communications unit 40 of the automated analyzer may be configured to buffer a plurality of automated analyzer status data messages for a given amount of time, and to concatenate the messages into one data packet, for example. This may lead to longer battery life of a handheld automated analyzer.

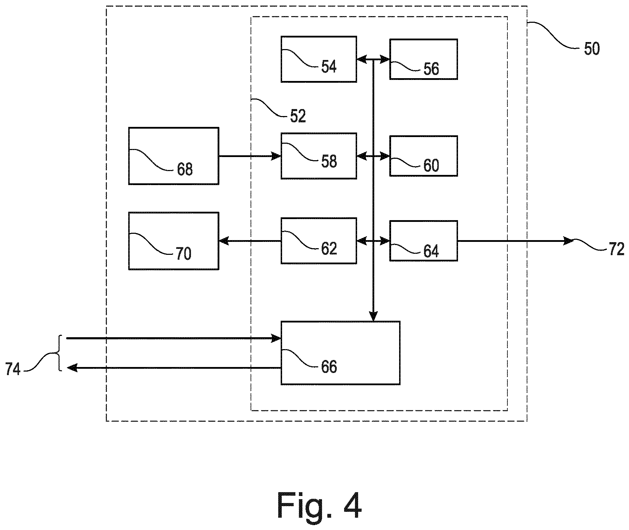

**FIG. 4** schematically illustrates an example of a server 50 configured to host a data processing agent.

In this example, the server 50 comprises a motherboard 52 comprising a random access memory 54, a read-only memory 56, a central processing unit 60, an input/output interface 58, a data storage unit 64 (such as a hard disk), a display interface 62, and a communication interface 66, however a skilled person will appreciate that many different types of server configuration can be provided with more or fewer modules having other functionality.

The data storage unit 64 of the server 50 comprises computer readable instructions which, when executed, instantiate a data processing agent for processing data from an automated analyzer of medical samples in the random access memory 54 of the server 50.

The communication interface 66 of the server is configured to interface with the communications network 16 of system for automated analyzer management. Automated analyzer status data from an automated analyzer P1 to P7 is received at the server 50 via the communication interface 66 of the server. Optionally, the automated analyzer status data is provided directly to the random access memory 54 by processing and analysis by the central processing unit. Optionally, the automated analyzer status data is written to the data storage unit 64 for subsequent analysis.

Optionally, the automated analyzer status data may be written (cached) to an external file store (not shown). On demand by the central processing unit 60, a request for the automated analyzer status data may be sent to an external file store over the communications network 16. The external file store may, pending authentication and authorization, transmit the automated analyzer status data to the server 50, where it may be subsequent processed and combined with annotation data.

Optionally, the annotation data may be written (cached) to an external file store (not shown). On demand by the central processing unit 60, a request for the data may be sent to an external file store over the communications network 16. The external file store may, pending authentication and authorization, transmit the annotation data to the server 50, where it may be subsequent processed and combined with annotation data.

The benefit of the foregoing two optional embodiments is that a large amount of automated analyzer status data and/or annotation data may be robustly stored until it needs to be processed. It is not essential that association of the annotation data and the automated analyzer status data is performed as data is received. Alternatively, the two types of data may be associated in a postprocessing step using, for example, timestamp data and/or lexical analysis of the received data.

Optionally, the annotation data and the automated analyzer status data is associated by the data processing agent immediately when it is received by the data processing agent.

The server 50 is also configured to receive one or more items of annotation data from an operator of an automated analyzer system at the data processing agent instantiated on the random access memory 54 of the server. The server 50 is optionally configured to index, and to store such annotation data on the data storage unit 64. Alternatively or in addition, the annotation data may be held in another device such as a portable computing device 26 that is connected to the communication network 16. Upon demand, or according to a regular timetable, remotely stored annotation data may be forwarded to the server 54 for storage and/or an external file store (not shown).

The annotation data will be discussed subsequently, but may comprise, for example, an unstructured text file containing information provided by one or more members of a POCT team about the operation of an automated analyzer system.

The data processing agent instantiated on the server 50 may receive one or more items of automated analyzer status data, and one or more items of annotation data. The data processing agent instantiated on the server 50 may associate one or more of the items of annotation data and automated analyzer status data according at least to techniques to be discussed subsequently.

The result of this association is an automated analyzer system data record. The automated analyzer system data record may be stored in the data storage unit 64 of the server for subsequent use. Alternatively or in addition, the automated analyzer system data record may be displayed to a user via display interface 62 and display screen 70. Alternatively or in addition, the automated analyzer system data record may be transmitted via the communications interface 66 over the communications network 16 to a portable computing device 26 and/or an external file store (not shown). This enables a member of the POCT team to access the associated automated analyzer status data and annotation data through one of many display modalities, and to browse the data to identify information about the technical operation of automated analyzers in the system for automated analyzer management.

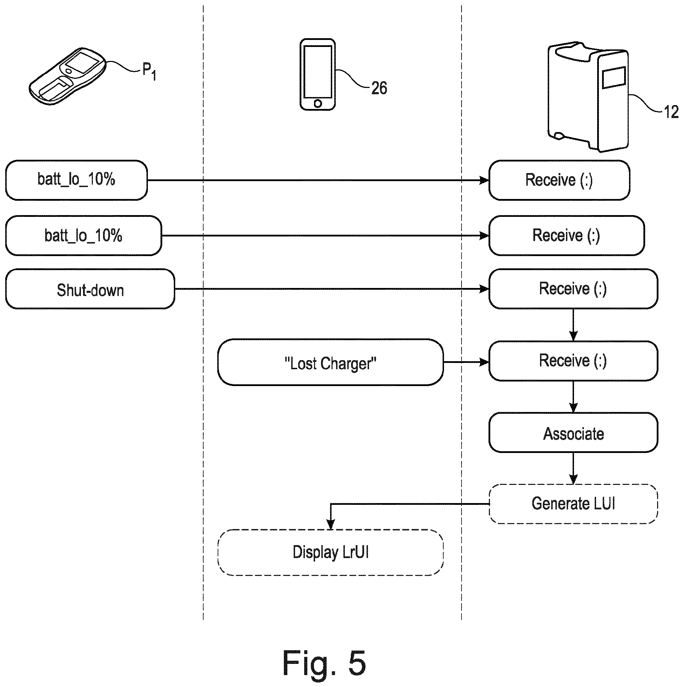

FIG. 5 schematically illustrates messaging in a use case in the system for automated analyzer management.

In FIG. 5, the messaging flow between an automated analyzer P1, a personal computing device 26, and a server 12 hosting a data processing agent according to the first aspect is illustrated. In the illustrated scenario, automated analyzer P1 transmits to signals indicating that the battery is depleting below a level of 10%, and eventually transmits a signal confirming that the automated analyzer P1 has shut down. Such a pattern of events may be typical, for example, when a member of a POCT team forgets to charge the battery of the automated analyzer P1.

Separately, either before, during, or after the succession of messages have been transmitted from the automated analyzer P1 to the server 12, a member of a POCT team may provide an unstructured text comment as an annotation (annotation data) referring to the fact that the charging device has gone missing. Such annotation data may alternatively be known as logbook data or diary data. The entry of such unstructured text comments into a text editor application executing on a personal computing device 26, as illustrated, is optional. Alternatively, the unstructured text comments may be hand-written into a paper diary and then scanned, or handwritten and then photographed using the personal computing device 26. Optical Character Recognition can be applied to such images to obtain unstructured annotation data. The unstructured text comments may be sent in the form of an email to a POCT team group email inbox. Optionally, the unstructured text comments may be entered as a diary entry in a computer calendar (for example, a Microsoft Outlook (TM) calendar). A skilled person will appreciate that many modalities for both recording and communicating the annotation data to the data processing agent exist.

In an embodiment, the annotation data is entered as an unstructured text entry in a text editor executing on a computing device. In an embodiment, the annotation data is entered as a semi-structured text entry into a text editor or database using an input form. In an embodiment, the annotation data is provided as a scanned image of a handwritten diary page.

In an embodiment, the annotation data is provided to the data processing agent via a Graphical User Interface (GUI). Optionally, the GUI may be instantiated on a display of a computer communicatively connected to the communications network 16 or the internal communications network 22. Optionally, the GUI may be instantiated on a display device of a POC analyzer. Optionally, the GUI may be instantiated on a personal computing device 16 such as a smartphone. Optionally, a user of the GUI provides the association of at least one item of annotation data with at least one item of automated analyzer status data using the GUI, or another user interface element.

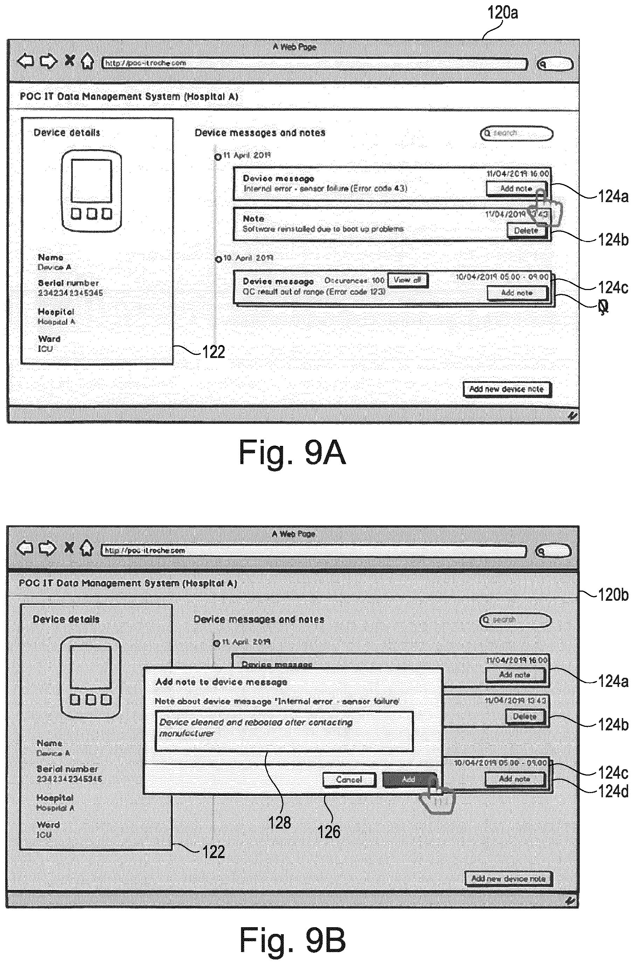

FIGS. 9A to 9D illustrate exemplary embodiments of an interactive GUI display for displaying instances of automated analyzer status data. As explained in the figure description of FIGS. 9A to 9D subsequently, the annotation data may be associated with the automated analyzer status data when a user operates the GUI.

In an embodiment, the annotation data is provided as a smart phone photograph image of a handwritten diary page. In an embodiment, the annotation data is captured using an electronic stylus, or from the screen of electronic notepad such as an iPad (TM) used in combination with an electronic stylus. In embodiments where the annotation data is provided as a handwritten annotation, the annotation data may be forwarded as an image of the original handwritten annotation.

Alternatively or in addition, the image of the handwritten annotation may be subject to optical character recognition (or text recognition) so that it may be converted into unstructured text before it is transmitted from a computing device to the data processing agent. Optionally, the data processing agent may perform optical character recognition on an original handwritten annotation to obtain unstructured text.

In an embodiment, the annotation data is spoken data captured using the microphone of a personal computing device 26 (such as a smartphone) or a dedicated dictaphone. Optionally, the spoken data may be forwarded to the data processing agent without pre-processing. In an embodiment, the spoken data may be processed using a voice recognition engine (such as Dragon Dictate (TM) or similar software) to obtain annotation data as unstructured text from the spoken data.

Medical professionals in a POCT team in a busy environment may prefer to make their notes (annotation data) concerning the operation of the automated analyzers over their shift using a dictaphone, and provide an entire audio file at the end of their shift.

Accordingly, in an embodiment the annotation data is generated by receiving an audio file and processing the audio file using an audio processing algorithm to extract individual comments over duration of time. Once extracted, individual audio comments are converted to unstructured text using a voice recognition engine. The individual audio comments as converted are then provided to the data processing agent. Alternatively, the data processing agent may perform the audio processing.

In an embodiment, the annotation data is video data captured with the video camera of a personal computing device 26 (such as a smart phone) or dedicated camera. Optionally, the video data may be forwarded to the data processing agent as a complete file.

In an embodiment, the data processing agent is configured to process the audio track of the video data using a voice recognition engine to extract unstructured text. The foregoing examples are provided for descriptive purposes only, and may be combined with each other, or other concepts without limitation.

Following reception of one or more items of automated analyzer status data and one or more items of annotation data, the data processing agent executing on the server 12 may associate one or more of the items of automated analyzer status data in a manner to be discussed. Optionally, the automated analyzer system data record generated by the data processing agent is stored either in the server 12 or transmitted to an external file storage system (not illustrated).

Optionally, the data processing agent generates a graphical user interface GUI comprising at least one of the items of automated analyzer status data and at least one of the items of the annotation data comprised in the automated analyzer system data record. The data processing agent may display the GUI so generated on a native display means connected to the server 12, alternatively or in addition the data processing agent may transmit the generated GUI to another device, such as the personal computing device 26, for display.

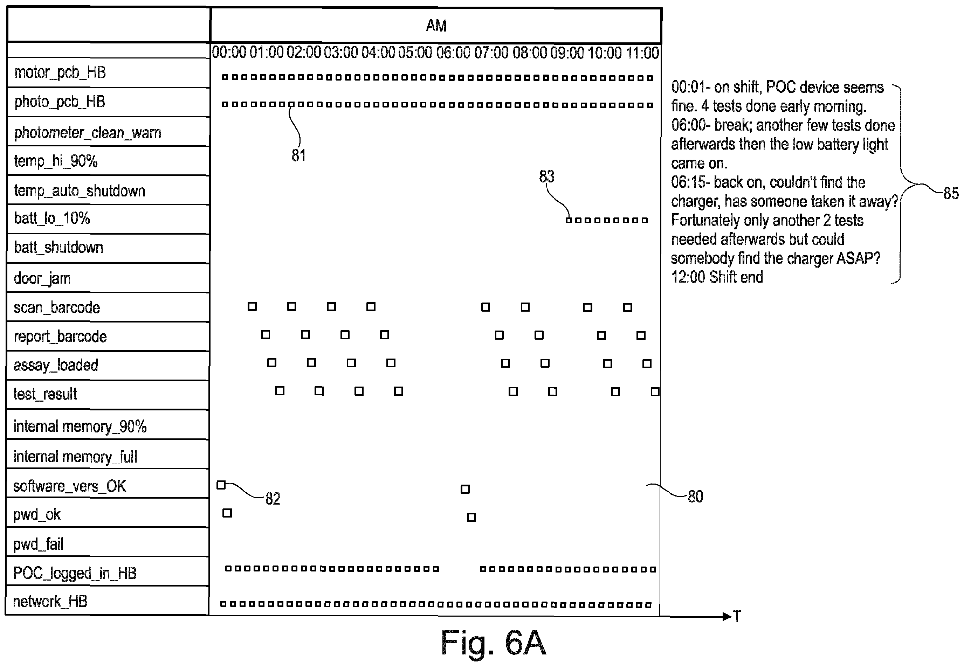

**FIG. 6A** schematically illustrates an example of a low-battery messaging scenario in a system for automated analyzer management. The example of FIG. 6A shows an example of the type of automated analyzer status data and automated annotation data that may be generated and received by the data processing agent.

The chart 80 represents the automated analyzer status data generated by the automated analyzer as it is generated in time T, with the chart 80 representing a twelve hour early morning shift. Repetitive heartbeat signal "photo_pcb_HB" indicates that the photometer is operating reliably, but generates a large number of messages in time. Upon initialization of the automated analyzer, a BIOS check ensures that the software (firmware) loaded onto the automated analyzer is of the correct version, indicated by event message 82, for example. Shortly afterwards, a user of the automated analyzer logs onto the device using a correct password, which is reported to the data processing agent using a further transmission of automated analyzer status data. Over the course of the shift, a sequence of eight analysis runs is performed, as shown by the repetitive sequence of automated analyzer status data reporting the scanning of a barcode, the reporting of a barcode the loading of an essay, and the provision of a test result. At around 09:30am, the automated analyzer begins to report a low battery alarm 83 in the form of automated analyzer status data transmitted to the data processing agent.

If a member of the POCT team responsible for the automated analyzer only had this data to assess the situation, it would not be clear why the automated analyzer was reporting the low battery alarm.

However, in this example a source of unstructured annotation data 85 was provided to the data processing agent using a text editing application capable of capturing short text comments in an interface of a personal communication device 26. From the unstructured annotation data 85, it becomes clear to the POCT team the reason that the automated analyzer is reporting a low battery alarm is that the department of the hospital 18 using the automated analyzer appears to have lost the charger of the device. Accordingly, contextual information that provides insight into the origin of the low battery alarm 83 can be obtained automatically at the data processing agent.

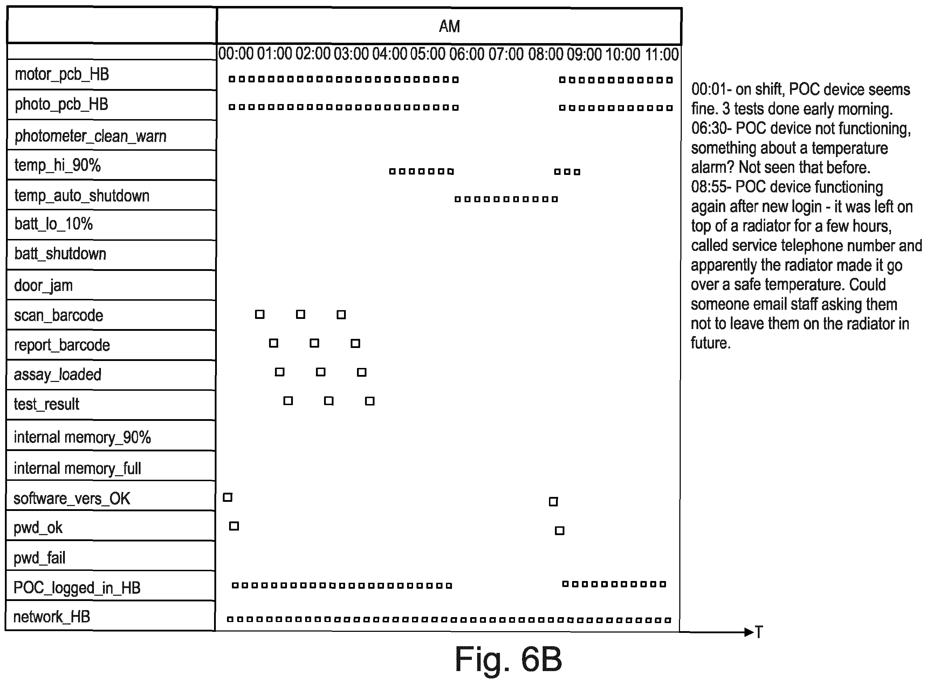

FIG. 6B schematically illustrates an example of a temperature anomaly messaging scenario in a system for automated analyzer management.

For example, the "motor_PCB" and "photo_PCB" heartbeat signals, configuration and password signals function as in the previous example. In this scenario, it is possible to for the data processing agent to deduce from the received automated analyzer status data that three diagnostic tests were performed in the early morning. The data processing agent receives a warning that the maximum operating temperature of the automated analyzer is close to being exceeded ("temp_hi_90%"), and subsequently that the automated analyzer has shut down ("temp_auto_shutdown"). However, apart from receiving these messages, the data processing agent is not able to provide actionable technical insight to the POCT team about why this particular automated analyzer has overheated.

The unstructured text comment provided according to 1 of the modalities discussed previously is also displayed in Fig. 6B. By associating the timing of the unstructured text comment entries with the timing of the automated analyzer status data, it immediately becomes clear that the automated analyzer was left on top of radiator and that this was explanation for the overheating alarms. Therefore, the data processing agent can use additional sources of annotation data to explain a previously unexplained technical condition of an automated analyzer.

According to an embodiment, the method further comprises receiving, at the data processing agent, a message from the automated analyzer of medical samples indicating that one or more items of automated analyzer status data should be supplemented with further data.

For example, the control and communications unit 45 of the automated analyzer 40 may comprise an analyzer rule engine that monitors the type and frequency of automated analyzer status data being generated by the automated analyzer 40 before it is communicated to the data processing agent. The analyzer rule engine of the automated analyzer 40 may determine that a given pattern, or trend, of automated analyzer status data requires further investigation. Therefore, the analyzer rule engine of the automated analyzer 40 may set a status flag in the automated analyzer status data before transmission of the automated analyzer status data to the data processing agent. Upon reception at the data processing agent, the data processing agent may identify that the status flag has been set by the automated analyzer. The data processing agent may transmit a demand for a POCT operator to provide further structured, semi-structured, or unstructured comment about the condition of the automated analyzer that set the status flag. In this way, an automated analyzer may identify that is operating in a partially unexplained set of technical circumstances requiring further elaboration by a POCT operator, and the POCT operator may be prompted to provide further elaboration by the automated analyzer via the data processing agent.

According to an embodiment, the method further comprises correlating, at the data processing agent, one or more items of automated analyzer status data with one or more items of annotation data, and performing, at the data processing agent, the association of one or more of the items of automated analyzer status data with one or more of the items of annotation data using the processing agent based on the correlation.

A skilled person will appreciate that the association of one or more items of automated analyzer status data with one or more items of annotation data may be performed in many different ways dependent on the type of annotation data provided, and on the particular combination of analyzer status data available. The following example illustrates one approach for associating unstructured text data with automated analyzer status data, but a skilled person could implement many other variations. The example technique is not essential.

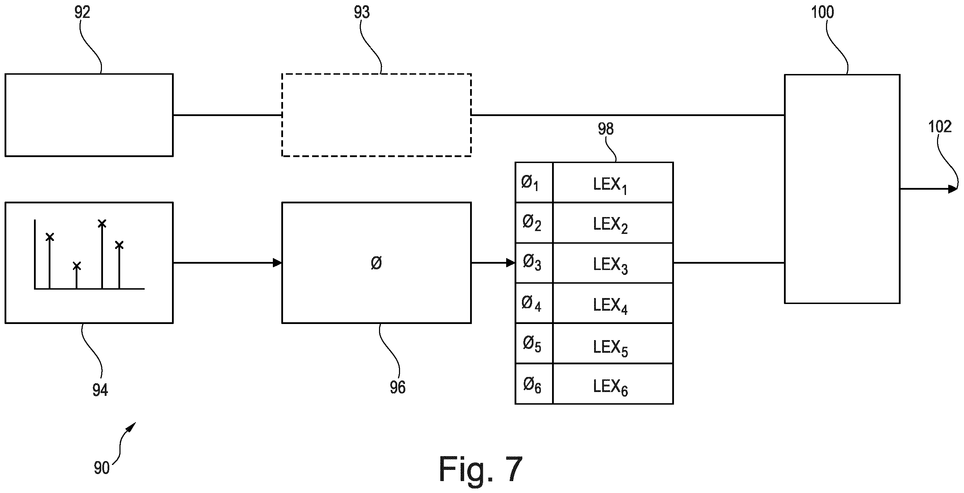

FIG. 7 schematically illustrates an example of a correlator for associating items of automated analyzer data with annotation data.

The correlator 90 illustrated in Figure 7 is, in an embodiment, implemented as a software engine executing within the data processing agent of the server 12. In another embodiment, the correlator may be implemented as a software engine in a remote cloud server, and provide correlated annotation data and automated analyzer status data to the data processing agent.

The correlator 90 may correlate annotation data and automated analyzer status data in real time (in other words, at the time that the respective data is received at the correlator).

Optionally, the annotation data and automated analyzer status data are correlated or associated based on a time stamp of when the annotation data and automated analyzer status data were created at the automated analyzer, and/or received at the data processing agent.

Optionally, the annotation data and automated analyzer status data are correlated or associated based on content analysis of the annotation data at the data processing agent, and a comparison with a plurality of automated analyzer status data received at the data processing agent.

Alternatively or in addition, the correlator may correlate annotation data and automated analyzer status data in a batch-run at a point in time after the time when the data was received from the automated analyzer.

The correlator 90 comprises an annotation data reception unit 92. The annotation data reception unit receives one or more items of annotation data generated, for example, by a POC technician according to one of the modalities discussed above (reception of unstructured or semi-structured text from a text application, OCR recognition of hand-written comments, recognition of comments from an electronic stylus, audio or video analysis, and the like).

The correlator 90 optionally comprises an annotation data categorization unit 93. Received automated analyzer status data is categorized based on, for example, time of receipt, category of message (concerning software configuration, security, authentication, test performance, hardware status, battery status, and the like). The categorization may, for example, be based on a prior semantic schema provided by manufacturer that links different types of automated analyzer status data to semantic concepts such as "low battery", "clean photometer".

The correlator 90 comprises an automated analyzer status data analysis engine 94. The function of the analysis engine 94 is to obtain, from a set or subset of the received automated analyzer status data, a pattern or indication of trends in the automated analyzer status data that indicate a prior indication of a semantically meaningful event (event data) connected to the automated analyzer, for example.

In an example, the analysis engine 94 obtains a histogram of the messages received over a given time window.

In other words, the automated analyzer status data analysis engine 94 functions to perform mathematical analysis, or statistical analysis, or signal processing on the received annotation data to, for example, identify significant trends in the data.

Optionally, the time window is configurable. The time window may be one year, one month, one week, one day, or may be linked to operational considerations such as a shift pattern. Optionally, the time window may be associated with a certified user of the automated analyzer.

The correlator 90 comprises a significance engine 96. The significance engine 96 receives event data from the data analysis engine 94. Turning briefly to the example of FIG 6A, a reader will appreciate that a statistical analysis of the automated analyzer status data may be skewed because some types of relatively insignificant automated analyzer status data may be transmitted frequently as a normal measure (such as a PCB heartbeat signal). On the other hand, significant automated analyzer data may only be transmitted once, for example a "shock" signal indicating that the automated analyzer has been dropped on the floor.

Accordingly, the purpose of the significance engine 96 is to filter the data from the data analysis engine 94 to prioritize significant automated analyzer status data, according to a prioritization function which, as a skilled person will appreciate, could be defined as a lookup table or in other ways.

The correlator 90 comprises a lexical transform engine 98. The lexical transform engine 98 obtains the prioritized analyzer status data from the significance engine 96, and performs a mapping of significant automated analyzer status data to corresponding lexical concept data. The lexical concept data is defined by a manufacturer of point-of-care equipment, for example. As an example, it provides the connection between unstructured text data (for example) entered as annotation data by a POCT operator, and the automated analyzer status data output by one or more automated analyzers, in an example. Each prioritization function ϕ represents a semantic concept such as "charge battery", "analyzer dropped", "memory full".

When automated analyzer status data is received by the correlator 90, the significance engine 96 identifies that a pattern of one or more items of automated analyzer status data may refer to such a semantic concept, and transmits an identifier referring to the semantic concept to the lexical transform engine 98.

The lexical transform engine 98 contains a data structure, or database, comprising at least one record for each semantic concept. Each record defines one or more words, word fragments, sentences and the like associated with a semantic concept, and likely to be present in associated unstructured comments provided in the annotation data by a POCT user.

The correlator 90 further comprises a comparison engine 100 configured to receive at least one record from the lexical transform engine and at least one item of annotation data from the annotation data reception unit 92 (optionally categorized by the annotation data categorization unit 93).

The comparison engine 100 isolates, in the annotation data, one or more items of relevant unstructured or semi structured text relevant to the semantic concept identified by the lexical analysis engine.

Once isolated, the items of relevant unstructured or semi structured text may be associated with one or more items of corresponding automated analyzer status data.

This enables an automated analyzer system data record to be generated, in which the relevant unstructured or semi structured text and associated corresponding automated analyzer status data are bound together.

Optionally, the automated analyzer system data record may be output and stored either in the server hosting the data processing agent, and/or in an external storage means.

Optionally, the automated analyzer system data record may be displayed on a graphical user interface.

Optionally, the automated analyzer system data record may be used to format the display of at least one item of automated analyzer status data on a graphical user interface to provide a clearer understanding of the technical status of a system of automated analyzers.

Optionally, the received automated analyzer status data may be categorized based on whether or not the automated analyzer has raised a flag or a rating for a specific item of automated analyzer status data requesting further elaboration of that data.

According to an embodiment, the method further comprises that the correlation is based on a time relationship between the generation of the one or more items of automated analyzer status data and the one or more items of annotation data; or wherein the correlation is based on one or more correlation rules related to the type of automated analyzer status data received at the data processing agent.

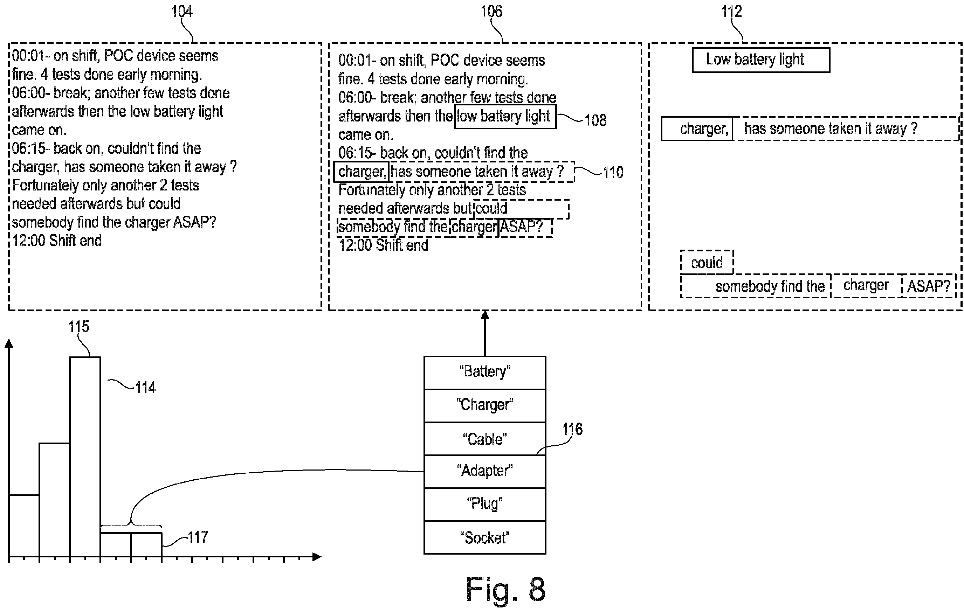

FIG. 8 schematically illustrates an example of correlating automated analyzer data with annotation data using a correlation rule set.

Record 104 illustrates unformatted unstructured annotation data that may be received, in a nonlimiting example, from a text input via a graphical user interface on a personal computing device 26.

Histogram 114, as generated by the analysis engine 94, shows a high incidence of automated analyzer status data type 115, which in this example refer to the "motor_PCB" handshake which it is preferred to de-emphasize, owing to the large number of messages. On the other hand, automated analyzer status data 117 refer to the "batt_lo_10%" annotation data. It is preferred to emphasize unstructured annotation data referring to concepts related to power issues.

Accordingly, the significance engine 94 detects that the "batt_lo_10%" is present, and should be emphasized.

The lexical transform engine 98 identifies that concepts relating to battery and power problems should be identified. A data record 116 in the lexical transform engine 98 is obtained using the output of the significance engine 94. The data record 116 provides textual samples of potential entries in unstructured text that could refer to the semantic concept indicated by the automated analyzer status data.

For example, the textual samples in the data record 116 relating to the "batt_lo_10%" annotation data comprise the text fragments "battery", "charger", "cable", "adapter", "plug", "socket".

A skilled person will appreciate that completely different textual samples would be provided in data record 116 for other semantic concepts related to the automated analyzer.

The comparison engine receives the original unstructured text data 104 and the data record 116 generated by the lexical transform engine. In one example, a text search of each textual sample in the data record 116 is performed over the unstructured text data 106 of the annotation data.

In a one case, words which are exact and/or close matches to words in the data record 116 are labelled. For example, the phrase "low battery light" 108 is labelled because the term "battery" occurs in data record 116.

In another case, the word "charger" is firstly identified in the unstructured text data 106 of the annotation data, and the labelling of the fragment is extended to encompass the entire clause attached to the word "charger".

As such, it is possible to identify close matches to text in the data record 116, and/or to extract an entire sentence or clause from the unstructured annotation data within which the text from the data record 116 occurs.

In one example, the output of the correlator 90 is a set of text fragments 112 that are associated with the automated analyzer status data received from one or more automated analyzers.

In an example, the output of the correlator 90 is provided directly on a graphical user interface as a representation of relevant text fragments from the annotation data.

In an example, the output of the correlator 90 is used to annotated representations of automated analyzer status data as will be discussed subsequently.

According to an embodiment, the method further comprises displaying a visual representation of the automated analyzer system data record on a graphical user interface, wherein the visual representation comprises a first item of automated analyzer status data.

As illustrated in FIG 4, for example, server 50 may comprise a display interface 62 and display screen 70. Therefore, the server 50 may render the visual representation of the automated analyzer system data record on display screen 70. However, it will be appreciated that many other ways of displaying the visual representation are possible.

In an embodiment, the server 50 may transmit the automated analyzer system data record to a personal computing device 26, such as a smart phone. The automated analyzer system data record may be displayed on the screen of the personal computing device 26.

In an embodiment, the server 50 may transmit the automated analyzer system data record to another personal computer on the communications network 16, so that the other personal computer on the communications network 16 can display the automated analyzer system data record.

In an embodiment, the server 50 may transmit the automated analyzer system data record to an automated analyzer P1 comprising a screen. Accordingly, a user of an automated analyzer P1 may view the automated analyzer system data record using an automated analyzer P1.

In an embodiment, the server 50 may host an intranet page or an Internet page upon which the automated analyzer system data record is displayed.

In an embodiment, the server 50 may transmit the automated analyzer system data record to an external storage device via the communications network 16. Another network device may connect to the external storage device via the communication network 16, access the automated analyzer system data record, and display it to user.

The automated analyzer system data record comprises at least one item of automated analyzer device status data, associated with one or more items of annotation data. Therefore, in a simple form, displaying the automated analyzer system data record comprises displaying the automated analyzer device status data in combination with the annotation data. Alternatively, a subset of the annotation data that is correlated to a subset of the automated analyzer status data by the correlation unit 90 may be displayed. In this way, a user only needs to view a greatly reduced proportion of the total amount of automated analyzer status data and annotation data in order to obtain relevant information about the operation of the automated analyzer.

According to an embodiment, the method further comprises prompting, via the graphical user interface, a user to provide unstructured comment data associated with the one or more items of automated analyzer status data, or the plurality of automated analyzer system data records, displayed on the graphical user interface, receiving, via an input interface, the unstructured comment data and associating the unstructured comment data with the one, or the plurality, of automated analyzer system data records.

FIG. 9A illustrates a first example of a GUI 120a. In the example of Figure 9A, the user can see comprehensive annotation data for a "Device A" in an Intensive Care Unit (ICU) of hospital A. As an example, the comprehensive annotation data functions as a combined logbook, which has been automatically assembled by a data processing agent according to the first aspect based on the one or more items of automated analyzer status data, and the one or more items of annotation data.

The logbook of FIG 9A comprises of device messages (events, or automated analyzer status data), and notes (annotation data) that were added by one of the users of the POC IT data management system.

The GUI 120a comprises a "device details" box 122 to inform a user of the GUI about which automated analyzer they are viewing. Optionally, the "device details" box 122 may comprise "live" information gathered from the automated analyzer status data by the data processing agent. For example, the "device details" box may discuss the level of consumable, or how many users are authorized to use the device in question.

The GUI 120a comprises a set of logbook entries 124a-d corresponding to automated analyzer system data records. As an example, one automated analyzer system data record is comprised of a displayed item of automated analyzer status data 124a ("Device message") and an associated item of annotation data 124b ("Note").

In the embodiment of FIG 9A, the box displaying the automated analyzer status data 124a contains a GUI button with the caption "Add note", although the caption and graphical nature of the GUI button are not essential. The GUI button is one form of a prompt to add annotation data to the automated analyzer status data 124a, although the prompt could be provided by a "pop-up" menu prompt, otherwise known as a "tool tip". Alternatively, the field for adding unstructured text could be provided directly into the "device message" box 124a in an open text field.

FIG. 9B illustrates an optional embodiment of a user being prompted, via the graphical user interface, to provide unstructured comment data associated with one or more items of automated analyzer status data. Upon actuation of the "add note" GUI button 124a, a pop-up box 126 is displayed to the user. Optionally, the color or transmittivity of the background to the pop-up box 126 may be changed to enhance focus on the foreground pop-up box 126. The pop-up box 126 comprises an unstructured text entry box. In this way, an item of automated analyzer status data 124a referring to a generated as a result of a "sensor failure" is annotated with annotation data comprising unstructured text stating "device cleaned and rebooted after contacting manufacturer". The automated analyzer status data and the annotation data provided thereby are, therefore, associated with each other.

Alternatively, or in combination, the GUI may display the result of an automatic correlation between automated analyzer status data and annotation data. Grouping of display items can be performed on the basis of timestamp information associated with an item of automated analyzer status data and an item of annotation data. For example, items of annotation data that were entered at similar times to the time at which automated analyzer status data messages were produced can be displayed in groups.

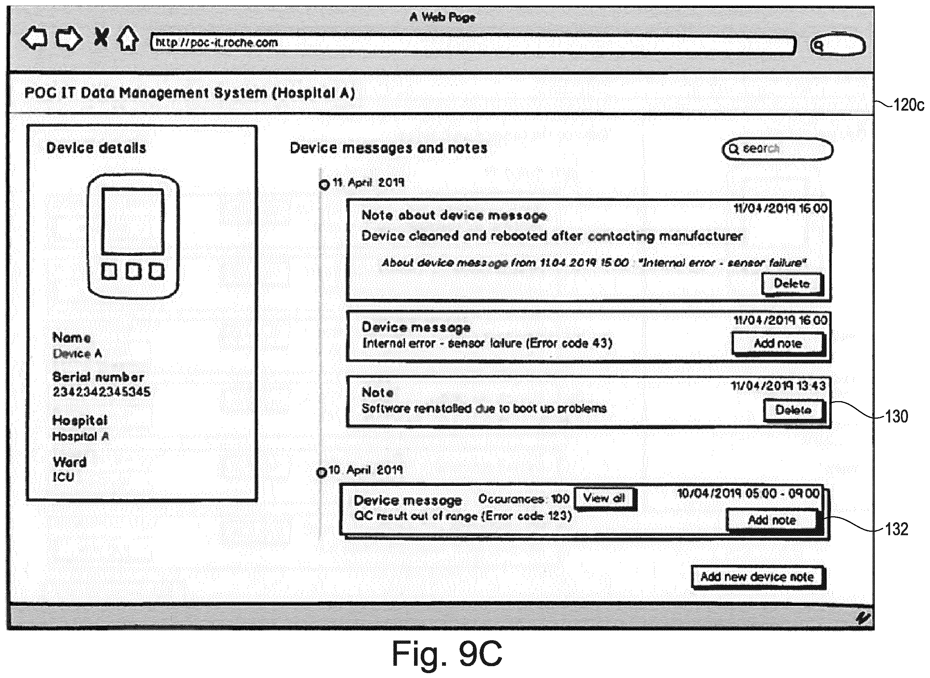

FIG. 9C illustrates that the unstructured annotation text has been added as a "Note" 130 in the GUI device message timeline. Therefore, the note is displayed together with information about which device message that the note has been made for.

According to an embodiment, the method further comprises displaying a visual representation of a plurality of automated analyzer system data records on a graphical user interface, wherein the visual representation comprises each record corresponding to an automated analyzer system data record of the same or a similar type.

FIG. 9C also illustrates a message stack 132, comprising a plurality of automated system data records collapsed into what appears to a user of the GUI to be a stacked appearance. Stacks of messages may automatically be created for automated analyzer status data that is created in large quantities. For example, the "heartbeat" signals illustrated in FIG. 6A and 6B may be collated and displayed as condensed stacks. In FIG. 9C, the message stack 133 comprises the automated system data record "QC result out of range" that, in the illustrated example, has occurred 100 times.

Accordingly, the action of "associating one or more items of automated analyzer status data with one or more of the items of annotation data using the data processing agent" may be performed partially, or completely, by a user interacting with the GUI. In an embodiment, the user may recognize from the device messages displayed on the GUI that a link between events is present, and enter unstructured text directly into the GUI.

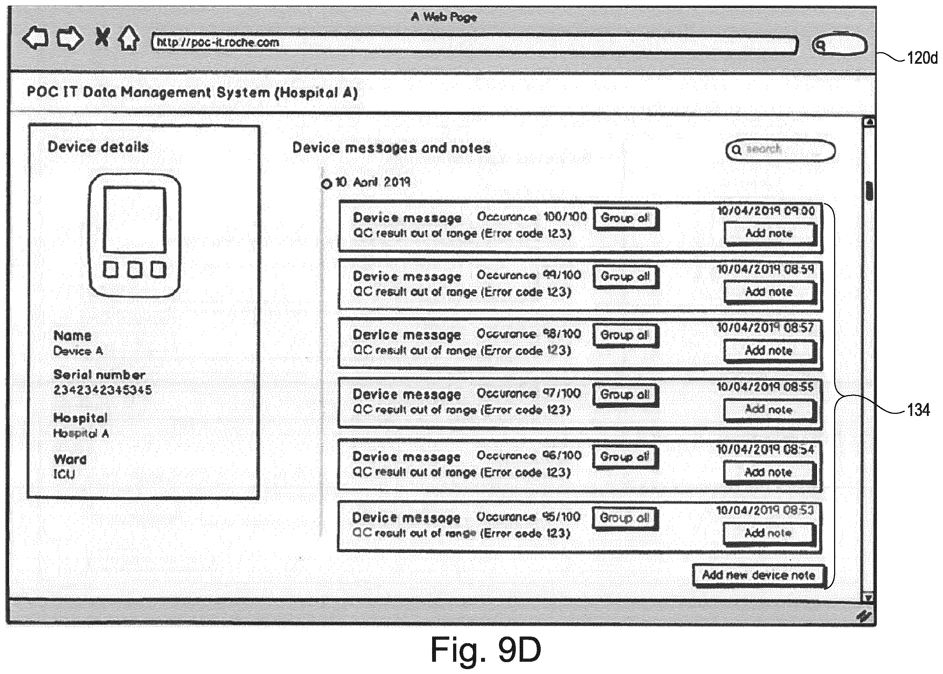

FIG. 9D illustrates a fourth example of the GUI, in which the plurality of automated system data records 130 are displayed in an expanded list 134. Optionally, the "Device message" entries in the stack display one or more of: the occurrence number of the message, the error code of the message, and the date and time of the message.

Optionally, when the stack of automated system data records has been expanded in the GUI, an "add note" option is available in each message box of the expanded stack 134. Accordingly, a user may provide a different unstructured text entry for each individual automated system data record displayed in the expanded GUI.

Adding unstructured data as annotation data to automated system data records is one option, but a GUI may enable a user to input annotation data as semi structured, or structured data, based on the context defined by received automated system data records.

For the purposes of this specification, unstructured data means free text, as in a diary entry. Semi-structured text is text that has been categorized, or which contains some binary or encoded answers such as "Yes/No". Structured data is generated entirely by controlled input means, such as via a GUI drop down box or series of check boxes, and affords no freedom to enter free text. One or more of the three options may be applicable to various clinical contexts and/or staff training levels.

The automated system data records can be used to predict the technical context that the machine is operating in (for example, "normal operation", "requires calibration", "battery low", "temperature alarm"). The technical context that the machine is operating in may be determined by the application of a display mask rule set. This is a logical engine which accounts for all (or a subset) of received automated system data records, and from the number of automated system data records received in each category, a GUI form is determined based on the context that the automated analyzer is determined to be operating in by the display mask rule set.

Optionally, the automated system data records applied to the display mask rule set are windowed based on time. In other words, automated system data records that are more than one minute, or one hour, or one day old are not used by the display mask rule set to determine which data fields to display to a user.

In this way, semi-structured data can be collected as annotation data using a GUI form which is more responsive to the technical operating context or condition of the automated analyzer, and many irrelevant data entry fields may be hidden from a user. This facilitates the entry of information, and reduces error. Optionally, the data entry fields may be one or more drop down menus, or check boxes.

According to an embodiment, the method further comprises analyzing, at the data processing agent, the one or more items of automated analyzer status data and/or the annotation data, determining, at the data processing agent, one or more data input fields to display to a user based on a display mask rule set, wherein the display mask rule set defines combinations of data input fields to be displayed to a user based on the received combination of automated analyzer status data, displaying, via the graphical user interface, a data input mask comprising one or more data input fields based on the received combination of automated analyzer status data, receiving, at the data processing agent, via an input interface, semi-structured data or structured data via the displayed data input mask, and associating the semi-structured data or structured data with one or more of the items of automated analyzer status data, and/or one or more of the items of annotation data, and optionally displaying the semi-structured data or structured data on the graphical user interface.

According to an embodiment, the method further comprises in that in the automated analyzer status data comprises messages relating to the condition of an automated analyzer, and optionally comprises battery status data, firmware configuration data of the automated analyzer, motor status data, log file data, network configuration data, temperature data, user identity data, password data, sensor data, assay result data, reset data, upgrade data, audit log data, local time data, or system time data.

According to an embodiment, the annotation data comprises unstructured text data, structured text data, scanned image data, video data, or audio data.

According to the second aspect, the apparatus 12 configured to host a data processing agent for processing data from an automated analyzer of medical samples comprises:
- a communications interface 66; and
- a processor 60 coupled to the communications interface and configured, in operation, to perform the functions of the data processing agent;
   wherein the communications interface 66 is configured to receive one or more items of automated analyzer status data transmitted from one or more automated analyzers in a medical sample analysis system to the data processing agent via a communications network, wherein the automated analyzer status data is generated by the one or more automated analyzers in response to an automated analyzer condition detected by a respective automated analyzer;
   wherein the communications interface 66 is configured to receive one or more items of annotation data at the data processing agent;
   wherein the processor 60 is configured to associate one or more of the items of automated analyzer status data with one or more of the items of annotation data using the data processing agent to thus generate an automated analyzer system data record; and
- wherein the processor 60 is configured to output, via the communications interface, an automated analyzer system data record from the data processing agent, wherein the automated analyzer system data record comprises one or more items of automated analyzer device status data associated with one or more of the items of annotation data.

As an example, the apparatus 12 of the second aspect may be implemented as a server 50.

According to a third aspect, there is provided a system for automated analyzer management comprising:
- one or more automated analyzers P1-P7 configured to analyze patient medical samples;
- an apparatus 12 configured to host a data processing agent for processing data from an automated analyzer of medical samples as defined in the second aspect, which, in operation, performs the method of the first aspect;
- a computing apparatus 24, 26 comprising a user interface; and
- a communication network 16 configured to communicatively connect the one or more automated analyzers, the computing device, and the apparatus configured to host a data processing agent;

wherein the one or more automated analyzers are configured to generate one or more items of automated analyzer status data, and the communication network is configured to communicate the one or more items of automated analyzer status data to the data processing agent hosted on the apparatus; and
wherein the computing apparatus is configured to communicate annotation data to the data processing agent hosted on the apparatus via the communication network.

According to a fourth aspect, there is provided a computer program element comprising computer-readable instructions for controlling an apparatus according to claim 12 which, when being executed by a processing unit of the apparatus, is adapted to perform the method steps of the first aspect.

According to a fifth aspect, there is provided a computer readable medium or signal having stored, or encoded thereon, the computer program element of the fourth aspect.

According to a supplementary aspect, there is provided an apparatus configured to host a data processing agent for processing data from an automated analyzer of medical samples in a medical sample analysis system, comprising:
- a communication means; and
- a processing means coupled to the communications means and configured, in operation, to perform the functions of the data processing agent;
   wherein the communication means is configured to receive one or more items of automated analyzer status data transmitted from one or more automated analyzers in a medical sample analysis system to the data processing agent via a communications network, wherein the automated analyzer status data is generated by the one or more automated analyzers in response to an automated analyzer condition detected by a respective automated analyzer;
   wherein the communications means is configured to receive one or more items of annotation data at the data processing agent;
   wherein the processing means is configured to associate one or more of the items of automated analyzer status data with one or more of the items of annotation data using the data processing agent to thus generate an automated analyzer system data record; and
- wherein the processing means is configured to output, via the communications interface, an automated analyzer system data record from the data processing agent, wherein the automated analyzer system data record comprises one or more items of automated analyzer device status data associated with one or more of the items of annotation data.

## Claims

1. A computer implemented method (30) for operating a data processing agent for processing data from an automated analyzer of medical samples (40) comprising:
- receiving (32) one or more items of automated analyzer status data transmitted from one or more automated analyzers in a medical sample analysis system to the data processing agent via a communications network, wherein the automated analyzer status data is generated by the one or more automated analyzers in response to an automated analyzer condition detected by a respective automated analyzer, and wherein the automated analyzer status data is generated by the automated analyzer in response to an operating condition inside the automated analyzer;
- receiving (34) one or more items of annotation data at the data processing agent from an operator;
- associating (36) one or more of the items of automated analyzer status data with the one or more items of annotation data using the data processing agent to provide a logical link between the one or more items of automated analyzer status data and the annotation data, to thus generate an automated analyzer system data record; and
- outputting (38) the automated analyzer system data record from the data processing agent, wherein the automated analyzer system data record comprises one or more items of automated analyzer device status data associated with one or more of the items of annotation data.

2. The computer-implemented method (30) according to claim 1, further comprising:
- detecting, at the data processing agent, that the one or more items of automated analyzer status data should be supplemented with annotation data based on an annotation rule set.

3. The computer-implemented method (30) according to claim 1, further comprising:
- receiving, at the data processing agent, a message from the automated analyzer of medical samples comprising a status flag set by the automated analyzer, indicating that one or more items of automated analyzer status data should be supplemented with annotation data.

4. The computer-implemented method (30) according to one of the preceding claims, further comprising:
- correlating, at the data processing agent, one or more items of automated analyzer status data with one or more items of annotation data, and
- performing, at the data processing agent, the association of one or more of the items of automated analyzer status data with one or more of the items of annotation data using the processing agent based on the correlation.

5. The computer-implemented method (30) according to dependent claim 4,
wherein the correlation is based on a time relationship between the generation of the one or more items of automated analyzer status data and the one or more items of annotation data; or wherein the correlation is based on one or more correlation rules of a correlation rule set, wherein the one or more correlation rules are related to the type of automated analyzer status data received at the data processing agent.

6. The computer-implemented method (30) according to one of the preceding claims, further comprising:
- displaying a visual representation of the automated analyzer system data record on a graphical user interface, wherein the visual representation comprises an item of automated analyzer status data, and an associated item of annotation data.

7. The computer-implemented method (30) according to one of the preceding claims, further comprising:
- displaying a visual representation of a plurality of automated analyzer system data records on a graphical user interface, wherein the visual representation comprises each record corresponding to an automated analyzer system data record of the same.

8. The computer-implemented method (30) according to one of claims 6 or 7, further comprising:
- prompting, via the graphical user interface, a user to provide unstructured comment data to be associated with the one or more items of automated analyzer status data, or the plurality of automated analyzer system data records, displayed on the graphical user interface;
- receiving, via an input interface, the unstructured comment data; and
- associating the unstructured comment data with the one, or the plurality, of automated analyzer system data records.

9. The computer-implemented method (30) according to one of the preceding claims, further comprising:
- analyzing, at the data processing agent, one or more automated system data records to determine a technical context that the automated analyzer is operating in;
- determining, at the data processing agent, one or more data input fields to display to a user based on a display mask rule set, wherein the display mask rule set defines combinations of data input fields to be displayed to a user based on the received combination of automated analyzer status data;
- displaying, via the graphical user interface, a data input mask comprising one or more data input fields based on the received combination of automated analyzer status data;
- receiving, at the data processing agent, via an input interface, semi-structured data or structured data via the displayed data input mask; and
- associating the semi-structured data or structured data with one or more of the items of automated analyzer status data, and/or one or more of the items of annotation data, and optionally displaying the semi-structured data or structured data on the graphical user interface.

10. The computer-implemented method (30) according to one of the preceding claims,
wherein the automated analyzer status data comprises messages relating to the condition of an automated analyzer, and optionally comprises battery status data, firmware configuration data of the automated analyzer, motor status data, log file data, network configuration data, temperature data, user identity data, password data, sensor data, assay result data, reset data, upgrade data, audit log data, local time data, or system time data.

11. The computer-implemented method (30) according to one of the preceding claims,
wherein the annotation data comprises unstructured text data, structured text data, scanned image data, video data, or audio data.

12. An apparatus (50) configured to host a data processing agent for processing data from an automated analyzer of medical samples, comprising:
- a communications interface (66); and
- a processor (60) coupled to the communications interface (66) and configured, in operation, to perform the functions of the data processing agent;
wherein the communications interface is configured to receive one or more items of automated analyzer status data transmitted from one or more automated analyzers in a medical sample analysis system to the data processing agent via a communications network, wherein the automated analyzer status data is generated by the one or more automated analyzers in response to an automated analyzer condition detected by a respective automated analyzer;
wherein the communications interface is configured to receive one or more items of annotation data from an operator at the data processing agent;
wherein the processor is configured to associate one or more of the items of automated analyzer status data with one or more of the items of annotation data using the data processing agent to thus generate an automated analyzer system data record; and
- wherein the processor is configured to output, via the communications interface, an automated analyzer system data record from the data processing agent, wherein the automated analyzer system data record comprises one or more items of automated analyzer device status data associated with one or more of the items of annotation data.

13. A system (10) for automated analyzer management comprising:
- one or more automated analyzers (P1-P7) configured to analyze patient medical samples;
- an apparatus (12) configured to host a data processing agent for processing data from an automated analyzer of medical samples as defined in claim 12 which, in operation, performs the method of claims 1 to 11;
- a computing apparatus (24, 26) comprising a user interface; and
- a communication network (16) configured to communicatively connect the one or more automated analyzers, the computing device, and the apparatus configured to host a data processing agent;
wherein the one or more automated analyzers are configured to generate one or more items of automated analyzer status data, and the communication network is configured to communicate the one or more items of automated analyzer status data to the data processing agent hosted on the apparatus; and
wherein the computing apparatus is configured to communicate annotation data to the data processing agent hosted on the apparatus via the communication network.

14. A computer program element comprising computer-readable instructions for controlling an apparatus according to claim 12 which, when being executed by a processing unit of the apparatus, is adapted to perform the method steps of one of claims 1 to 11.

15. A computer readable medium or signal having stored, or encoded thereon, the computer program element of claim 14.

## Patentansprüche

1. Computerimplementiertes Verfahren (30) zum Betreiben eines Datenverarbeitungsmittels zum Verarbeiten von Daten aus einem automatisierten Analysator von medizinischen Proben (40), umfassend:
- Empfangen (32) eines oder mehrerer Elemente von Daten zum Status des automatisierten Analysators, die über ein Kommunikationsnetzwerk von einem oder mehreren automatisierten Analysator(en) in einem Analysesystem für medizinische Proben an das Datenverarbeitungsmittel übertragen wurden, wobei die Daten zum Status des automatisierten Analysators von dem einen oder den mehreren automatisierten Analysator(en) als Reaktion auf einen Zustand des automatisierten Analysators, der von einem entsprechenden automatisierten Analysator erfasst wurde, erzeugt werden und wobei die Daten zum Status des automatisierten Analysators von dem automatisierten Analysator als Reaktion auf einen Betriebszustand in dem automatisierten Analysator erzeugt werden;
- Empfangen (34) eines oder mehrerer Elemente von Anmerkungsdaten am Datenverarbeitungsmittel von einem Operator;
- Inverbindungbringen (36) eines oder mehrerer der Elemente von Daten zum Status des automatisierten Analysators mit dem einen oder den mehreren Elementen von Anmerkungsdaten unter Verwendung des Datenverarbeitungsmittels, um eine logische Verknüpfung zwischen dem einen oder den mehreren Elementen von Daten zum Status des automatisierten Analysators und den Anmerkungsdaten bereitzustellen, um so einen Datensatz zu dem automatisierten Analysatorsystem zu erzeugen; und
- Ausgeben (38) des Datensatzes zu dem automatisierten Analysatorsystem aus dem Datenverarbeitungsmittel, wobei der Datensatz zu dem automatisierten Analysatorsystem ein oder mehrere Elemente von Daten zum Status der automatisierten Analysatorvorrichtung umfasst, die mit einem oder mehreren der Elemente von Anmerkungsdaten in Verbindung stehen.

2. Computerimplementiertes Verfahren (30) nach Anspruch 1, ferner umfassend:
- Erfassen, dass das eine oder die mehreren Elemente von Daten zum Status des automatisierten Analysators basierend auf einem Anmerkungsregelsatz mit Anmerkungsdaten ergänzt werden sollten, an dem Datenverarbeitungsmittel.

3. Computerimplementiertes Verfahren (30) nach Anspruch 1, ferner umfassend:
- Empfangen einer Mitteilung von dem automatisierten Analysator für medizinische Proben, die ein von dem automatisierten Analysator gesetztes Statusflag umfasst, das angibt, dass eines oder mehrere Elemente von Daten zum Status des automatisierten Analysators mit Anmerkungsdaten ergänzt werden sollten, an dem Datenverarbeitungsmittel.

4. Computerimplementiertes Verfahren (30) nach einem der vorstehenden Ansprüche, ferner umfassend:
- Korrelieren eines oder mehrerer Elemente von Daten zum Status des automatisierten Analysators mit einem oder mehreren Elementen von Anmerkungsdaten an dem Datenverarbeitungsmittel und
- Durchführen des Inverbindungbringens eines oder mehrerer der Elemente von Daten zum Status des automatisierten Analysators mit einem oder mehreren der Elemente von Anmerkungsdaten unter Verwendung des Verarbeitungsmittels basierend auf der Korrelation an dem Datenverarbeitungsmittel.

5. Computerimplementiertes Verfahren (30) nach dem abhängigen Anspruch 4,
wobei die Korrelation auf einer Zeitbeziehung zwischen der Erzeugung des einen oder der mehreren Elemente von Daten zum Status des automatisierten Analysators und des einen oder der mehreren Elemente von Anmerkungsdaten basiert oder wobei die Korrelation auf einer oder mehreren Korrelationsregeln eines Korrelationsregelsatzes basiert, wobei sich die eine oder mehreren Korrelationsregeln auf den Typ von Daten zum Status des automatisierten Analysators, die an dem Datenverarbeitungsmittel empfangen werden, beziehen.

6. Computerimplementiertes Verfahren (30) nach einem der vorstehenden Ansprüche, ferner umfassend:
- Anzeigen einer visuellen Darstellung des Datensatzes zu dem automatisierten Analysatorsystem auf einer graphischen Benutzerschnittstelle, wobei die visuelle Darstellung ein Element von Daten zum Status des automatisierten Analysators und ein damit verbundenes Element von Anmerkungsdaten umfasst.

7. Computerimplementiertes Verfahren (30) nach einem der vorstehenden Ansprüche, ferner umfassend:
- Anzeigen einer visuellen Darstellung einer Vielzahl von Datensätzen zu dem automatisierten Analysatorsystem auf einer graphischen Benutzerschnittstelle, wobei die visuelle Darstellung jeden Satz, der einem Datensatz zu dem automatisierten Analysatorsystem desselben entspricht, umfasst.

8. Computerimplementiertes Verfahren (30) nach einem der Ansprüche 6 oder 7, ferner umfassend:
- Auffordern eines Benutzers über die graphische Benutzerschnittstelle, unstrukturierte Kommentardaten, die mit dem einen oder den mehreren Elementen von Daten zum Status des automatisierten Analysators in Verbindung zu bringen sind, oder die Vielzahl von Datensätzen zu dem automatisierten Analysatorsystem, die auf der graphischen Benutzerschnittstelle angezeigt werden, bereitzustellen;
- Empfangen der unstrukturierten Kommentardaten über eine Eingabeschnittstelle und
- Inverbindungbringen der unstrukturierten Kommentardaten mit dem einen Datensatz oder der Vielzahl von Datensätzen zu dem automatisierten Analysatorsystem.

9. Computerimplementiertes Verfahren (30) nach einem der vorstehenden Ansprüche, ferner umfassend:
- Analysieren eines Datensatzes oder mehrerer Datensätze zu einem automatisierten System an dem Datenverarbeitungsmittel, um einen technischen Zusammenhang zu bestimmen, in dem der automatisierte Analysator arbeitet;
- Bestimmen eines oder mehrerer Dateneingabefelder, die einem Benutzer anzuzeigen sind, basierend auf einem Anzeigemaskenregelsatz an dem Datenverarbeitungsmittel, wobei der Anzeigemaskenregelsatz Kombinationen von Dateneingabefeldern definiert, die einem Benutzer basierend auf der empfangenen Kombination von Daten zum Status des automatisierten Analysators angezeigt werden sollen;
- Anzeigen einer Dateneingabemaske, die ein oder mehrere Dateneingabefelder umfasst, basierend auf der empfangenen Kombination von Daten zum Status des automatisierten Analysators über die graphische Benutzerschnittstelle,
- Empfangen von halb strukturierten Daten oder strukturierten Daten über die angezeigte Dateneingabemaske an dem Datenverarbeitungsmittel über die Eingabeschnittstelle und
- Inverbindungbringen der halb strukturierten Daten oder strukturierten Daten mit einem oder mehreren der Elemente von Daten zum Status des automatisierten Analysators und/oder einem oder mehreren der Elemente von Anmerkungsdaten und gegebenenfalls Anzeigen der halb strukturierten Daten oder strukturierten Daten auf der graphischen Benutzerschnittstelle.

10. Computerimplementiertes Verfahren (30) nach einem der vorstehenden Ansprüche,
wobei die Daten zum Status des automatisierten Analysators Mitteilungen bezüglich des Zustands eines automatisierten Analysators umfassen und gegebenenfalls Batteriestatusdaten, Firmwarekonfigurationsdaten des automatisierten Analysators, Motorstatusdaten, Protokolldateidaten, Netzwerkkonfigurationsdaten, Temperaturdaten, Benutzeridentitätsdaten, Passwortdaten, Sensordaten, Assayergebnisdaten, Rückstelldaten, Aktualisierungsdaten, Revisionsprotokolldaten, lokale Zeitdaten oder Systemzeitdaten umfassen.

11. Computerimplementiertes Verfahren (30) nach einem der vorstehenden Ansprüche,
wobei die Anmerkungsdaten unstrukturierte Textdaten, strukturierte Textdaten, gescannte Bilddaten, Videodaten oder Audiodaten umfassen.

12. Apparat (50), der zum Hosten eines Datenverarbeitungsmittels zum Verarbeiten von Daten aus einem automatisierten Analysator für medizinische Proben ausgebildet ist, umfassend:
- eine Kommunikationsschnittstelle (66) und
- einen Prozessor (60), der an die Kommunikationsschnittstelle (66) gekoppelt und dazu ausgebildet ist, während des Betriebes die Funktionen des Datenverarbeitungsmittels auszuführen;
wobei die Kommunikationsschnittstelle zum Empfangen eines oder mehrerer Elemente von Daten zum Status des automatisierten Analysators, die über ein Kommunikationsnetzwerk von einem oder mehreren automatisierten Analysator(en) in einem Analysesystem für medizinische Proben an das Datenverarbeitungsmittel übertragen wurden, ausgebildet ist, wobei die Daten zum Status des automatisierten Analysators von dem einen oder den mehreren automatisierten Analysator(en) als Reaktion auf einen Zustand des automatisierten Analysators, der von einem entsprechenden automatisierten Analysator erfasst wurde, erzeugt werden;
wobei die Kommunikationsschnittstelle zum Empfangen eines oder mehrerer Elemente von Anmerkungsdaten von einem Operator am Datenverarbeitungsmittel ausgebildet ist;
wobei der Prozessor zum Inverbindungbringen eines oder mehrerer der Elemente von Daten zum Status des automatisierten Analysators mit einem oder mehreren Elementen von Anmerkungsdaten unter Verwendung des Datenverarbeitungsmittels ausgebildet ist, um so einen Datensatz zu dem automatisierten Analysatorsystem zu erzeugen; und
- wobei der Prozessor zum Ausgeben eines Datensatzes zu dem automatisierten Analysatorsystem aus dem Datenverarbeitungsmittel über die Kommunikationsschnittstelle ausgebildet ist, wobei der Datensatz zu dem automatisierten Analysatorsystem ein oder mehrere Elemente von Daten zum Status der automatisierten Analysatorvorrichtung umfasst, die mit einem oder mehreren der Elemente von Anmerkungsdaten in Verbindung stehen.

13. System (10) zur Verwaltung eines automatisierten Analysators, umfassend:
- einen oder mehrere automatisierte Analysatoren (P1-P7), die zum Analysieren von medizinischen Proben von Patienten ausgebildet sind;
- einen Apparat (12), der zum Hosten eines Datenverarbeitungsmittels zum Verarbeiten von Daten aus einem automatisierten Analysator für medizinische Proben, wie in Anspruch 12 definiert, ausgebildet ist, der während des Betriebes das Verfahren nach den Ansprüchen 1 bis 11 ausführt;
- einen Rechenapparat (24, 26), der eine Benutzerschnittstelle umfasst; und
- ein Kommunikationsnetzwerk (16), das dazu ausgebildet ist, den einen oder die mehreren automatisierten Analysatoren, die Rechenvorrichtung und den Apparat, der zum Hosten eines Datenverarbeitungsmittels ausgebildet ist, kommunikationsfähig zu verbinden;
wobei der eine oder die mehreren automatisierten Analysatoren zum Erzeugen eines oder mehrerer Elemente von Daten zum Status des automatisierten Analysators ausgebildet sind und das Kommunikationsnetzwerk zum Übermitteln des einen oder der mehreren Elemente von Daten zum Status des automatisierten Analysators an das von dem Apparat gehostete Datenverarbeitungsmittel ausgebildet ist; und
wobei der Rechenapparat zum Übermitteln von Anmerkungsdaten an das von dem Apparat gehostete Datenverarbeitungsmittel über das Kommunikationsnetzwerk ausgebildet ist.

14. Computerprogrammelement, umfassend computerlesbare Anweisungen zum Steuern eines Apparates nach Anspruch 12, die, wenn sie von einer Verarbeitungseinheit des Apparates ausgeführt werden, zum Durchführen der Verfahrensschritte nach einem der Ansprüche 1 bis 11 ausgelegt sind.

15. Computerlesbares Medium oder Signal, auf dem das Computerprogrammelement nach Anspruch 14 gespeichert oder verschlüsselt ist.

## Revendications

1. Procédé mis en oeuvre par ordinateur (30) pour faire fonctionner un agent de traitement de données pour traiter des données à partir d'un analyseur automatisé d'échantillons médicaux (40) comprenant :
- la réception (32) d'un ou de plusieurs éléments de données de statut d'analyseur automatisé transmis à partir d'un ou de plusieurs analyseurs automatisés dans un système d'analyse d'échantillons médicaux à l'agent de traitement de données par le biais d'un réseau de communication, les données de statut d'analyseur automatisé étant générées par le ou les analyseurs automatisés en réponse à un état d'analyseur automatisé détecté par un analyseur automatisé respectif, et les données de statut d'analyseur automatisé étant générées par l'analyseur automatisé en réponse à un état de fonctionnement à l'intérieur de l'analyseur automatisé ;
- la réception (34) d'un ou de plusieurs éléments de données d'annotation au niveau de l'agent de traitement de données à partir d'un opérateur ;
- l'association (36) d'un ou de plusieurs des éléments de données de statut d'analyseur automatisé au ou aux éléments de données d'annotation en utilisant l'agent de traitement de données pour fournir un lien logique entre le ou les éléments de données de statut d'analyseur automatisé et les données d'annotation, pour ainsi générer un enregistrement de données de système d'analyseur automatisé ; et
- l'émission (38) de l'enregistrement de données de système d'analyseur automatisé à partir de l'agent de traitement de données, l'enregistrement de données de système d'analyseur automatisé comprenant un ou plusieurs éléments de données de statut de dispositif d'analyseur automatisé associés à un ou plusieurs des éléments de données d'annotation.

2. Procédé mis en oeuvre par ordinateur (30) selon la revendication 1, comprenant en outre :
- la détection, au niveau de l'agent de traitement de données, que le ou les éléments de données de statut d'analyseur automatisé doivent être complétés par des données d'annotation en se basant sur un ensemble de règles d'annotation.

3. Procédé mis en oeuvre par ordinateur (30) selon la revendication 1, comprenant en outre :
- la réception, au niveau de l'agent de traitement de données, d'un message à partir de l'analyseur automatisé d'échantillons médicaux comprenant un ensemble de marqueurs de statut par l'analyseur automatisé, indiquant que le ou les éléments de données de statut d'analyseur automatisé doivent être complétés par des données d'annotation.

4. Procédé mis en oeuvre par ordinateur (30) selon l'une des revendications précédentes, comprenant en outre :
- la corrélation, au niveau de l'agent de traitement de données, d'un ou de plusieurs éléments de données de statut d'analyseur automatisé avec un ou plusieurs éléments de données d'annotation, et
- la réalisation, au niveau de l'agent de traitement de données, de l'association d'un ou de plusieurs des éléments de données de statut d'analyseur automatisé avec un ou plusieurs des éléments de données d'annotation en utilisant l'agent de traitement en se basant sur la corrélation.

5. Procédé mis en oeuvre par ordinateur (30) selon la revendication dépendante 4,
dans lequel la corrélation est basée sur une relation temporelle entre la génération du ou des éléments de données de statut d'analyseur automatisé et le ou les éléments de données d'annotation ; ou dans lequel la corrélation est basée sur une ou plusieurs règles de corrélation d'un ensemble de règles de corrélation, dans lequel la ou les règles de corrélation sont liées au type de données de statut d'analyseur automatisé reçues au niveau de l'agent de traitement de données.

6. Procédé mis en oeuvre par ordinateur (30) selon l'une des revendications précédentes, comprenant en outre :
- l'affichage d'une représentation visuelle de l'enregistrement de données de système d'analyseur automatisé sur une interface utilisateur graphique, la représentation visuelle comprenant un élément de données de statut d'analyseur automatisé et un élément associé de données d'annotation.

7. Procédé mis en oeuvre par ordinateur (30) selon l'une des revendications précédentes, comprenant en outre :
- l'affichage d'une représentation visuelle d'une pluralité d'enregistrements de données de système d'analyseur automatisé sur une interface utilisateur graphique, la représentation visuelle comprenant chaque enregistrement correspondant à un enregistrement de données de système d'analyseur automatisé du même type.

8. Procédé mis en oeuvre par ordinateur (30) selon l'une des revendications 6 ou 7, comprenant en outre :
- l'invite, par le biais de l'interface utilisateur graphique, d'un utilisateur à fournir des données de commentaires non structurées à associer au ou aux éléments de données de statut d'analyseur automatisé, ou à la pluralité d'enregistrements de données de système d'analyseur automatisé, affichés sur l'interface utilisateur graphique ;
- la réception, par le biais d'une interface de saisie, des données de commentaires non structurées ; et
- l'association des données de commentaires non structurées 1 ou à la pluralité des enregistrements de données de système d'analyseur automatisé.

9. Procédé mis en oeuvre par ordinateur (30) selon l'une des revendications précédentes, comprenant en outre :
- l'analyse, au niveau de l'agent de traitement de données, d'un ou plusieurs enregistrements de données de système automatisé pour déterminer un contexte technique dans lequel l'analyseur automatisé fonctionne ;
- la détermination, au niveau de l'agent de traitement de données, d'un ou de plusieurs champs de saisie de données à afficher à un utilisateur en se basant sur un ensemble de règles de masque d'affichage, l'ensemble de règles de masque d'affichage définissant des combinaisons de champs de saisie de données à afficher à un utilisateur en se basant sur la combinaison reçue de données de statut d'analyseur automatisé ;
- l'affichage, par le biais de l'interface utilisateur graphique, d'un masque de données comprenant un ou plusieurs champs de saisie de données en se basant sur la combinaison reçue de données de statut d'analyseur automatisé ;
- la réception, au niveau de l'agent de traitement de données, par le biais d'une interface de saisie, de données semi-structurées ou de données structurées par le biais du masque de saisie de données affiché ; et
- l'association des données semi-structurées ou des données structurées à un ou plusieurs des éléments de données de statut d'analyse automatisée, et/ou un ou plusieurs des éléments de données d'annotation, et éventuellement l'affichage des données semi-structurées ou des données structurées sur l'interface utilisateur graphique.

10. Procédé mis en oeuvre par ordinateur (30) selon l'une des revendications précédentes,
dans lequel les données de statut d'analyseur automatisé comprennent des messages concernant l'état d'un analyseur automatisé, et éventuellement comprennent des données de statut de batterie, des données de configuration de micrologiciel de l'analyseur automatisé, des données de statut de moteur, des données de fichier journal, des données de configuration de réseau, des données de température, des données d'identité d'utilisateur, des données de mot de passe, des données de capteur, des données de résultats d'analyse, des données de réinitialisation, des données de mise à jour, des données de journal d'audit, des données d'heure locale ou des données d'heure système.

11. Procédé mis en oeuvre par ordinateur (30) selon l'une des revendications précédentes,
dans lequel les données d'annotation comprennent des données de texte non structurées, des données de texte structurées, des données d'images scannées, des données vidéo ou des données audio.

12. Appareil (50) configuré pour héberger un agent de traitement de données pour traiter des données à partir d'un analyseur automatisé d'échantillons médicaux, comprenant :
- une interface de communication (66) ; et
- un processeur (60) couplé à l'interface de communication (66) et configuré, en fonctionnement, pour réaliser les fonctions de l'agent de traitement de données ;
dans lequel l'interface de communication est configurée pour recevoir un ou plusieurs éléments de données de statut d'analyseur automatisé transmis à partir d'un ou de plusieurs analyseurs automatisés dans un système d'analyse d'échantillons médicaux à l'agent de traitement de données par le biais d'un réseau de communication, dans lequel les données de statut d'analyseur automatisé sont générées par le ou les analyseurs automatisés en réponse à un état d'analyseur automatisé détecté par un analyseur automatisé respectif ;
dans lequel l'interface de communication est configurée pour recevoir un ou plusieurs éléments de données d'annotation à partir d'un opérateur au niveau de l'agent de traitement de données ;
dans lequel le processeur est configuré pour associer un ou plusieurs des éléments de données de statut d'analyseur automatisé à un ou plusieurs des éléments de données d'annotation en utilisant l'agent de traitement de données pour ainsi générer un enregistrement de données de système d'analyseur automatisé ; et
- dans lequel le processeur est configuré pour émettre, par le biais de l'interface de communication, un enregistrement de données de système d'analyseur automatisé à partir de l'agent de traitement de données, dans lequel l'enregistrement de données de système d'analyseur automatisé comprend un ou plusieurs éléments de données de statut de dispositif d'analyseur automatisé associés à un ou plusieurs des éléments de données d'annotation.

13. Système (10) de gestion d'analyseur automatisé comprenant :
- un ou plusieurs analyseurs automatisés (P1 à P7) configurés pour analyser des échantillons médicaux de patients ;
- un appareil (12) configuré pour héberger un agent de traitement de données pour traiter des données à partir d'un analyseur automatisé d'échantillons médicaux tel que défini dans la revendication 12 qui, en fonctionnement, réalise le procédé selon les revendications 1 à 11 ;
- un appareil de calcul (24, 26) comprenant une interface utilisateur ; et
- un réseau de communication (16) configuré pour connecter en communication le ou les analyseurs automatisés, le dispositif de calcul et l'appareil configuré pour héberger un agent de traitement de données ;
dans lequel le ou les analyseurs automatisés sont configurés pour générer un ou plusieurs éléments de données de statut d'analyseur automatisé, et le réseau de communication est configuré pour communiquer le ou les éléments de données de statut d'analyseur automatisé à l'agent de traitement de données hébergé sur l'appareil ; et
dans lequel l'appareil de calcul est configuré pour communiquer des données d'annotation à l'agent de traitement de données hébergé sur l'appareil par le biais du réseau de communication.

14. Élément de programme informatique comprenant des instructions lisibles par ordinateur pour commander un appareil selon la revendication 12 qui, lorsqu'il est exécuté par une unité de traitement de l'appareil, est adapté pour réaliser les étapes du procédé selon l'une des revendications 1 à 11.

15. Support ou signal lisible par ordinateur sur lequel est stocké ou codé l'élément de programme informatique selon la revendication 14.
